(19) Europäisches Patentamt
European Patent Office
Office européen des brevets

(11) EP 1 327 448 A1

(12) EUROPEAN PATENT APPLICATION
published in accordance with Art. 158(3) EPC

(43) Date of publication:
16.07.2003 Bulletin 2003/29

(21) Application number: 01967672.5

(22) Date of filing: 12.09.2001

(51) Int Cl.[7]: **A61K 31/737**, A61K 35/80, A61P 43/00, A61P 1/16, A61P 3/08, A61P 3/06, A61P 35/00, A61P 31/18, A61P 1/04, A23L 2/52, A23L 1/29, A23K 1/16, C08B 37/00

(86) International application number:
PCT/JP01/07894

(87) International publication number:
WO 02/022140 (21.03.2002 Gazette 2002/12)

(84) Designated Contracting States:
AT BE CH CY DE DK ES FI FR GB GR IE IT LI LU MC NL PT SE TR
Designated Extension States:
AL LT LV MK RO SI

(30) Priority: 13.09.2000 JP 2000278712
27.09.2000 JP 2000295077
09.11.2000 JP 2000342224
13.12.2000 JP 2000379313
25.04.2001 JP 2001128295
13.06.2001 JP 2001179335

(71) Applicant: TAKARA BIO INC.
Otsu-shi, Shiga 520-2193 (JP)

(72) Inventors:
- NISHIYAMA, Eiji
Moriyama-shi, Shiga 524-0102 (JP)
- SAGAWA, Hiroaki
Kusatsu-shi, Shiga 525-0025 (JP)
- HINO, Fumitsugu
Kusatsu-shi, Shiga 525-0028 (JP)
- MORIHARA, Etsuko
Otsu-shi, Shiga 520-2141 (JP)
- SAKAI, Takeshi
Hirosaki-shi, Aomori 036-8183 (JP)
- OYASHIKI, Haruo
Otsu-shi, Shiga 520-2276 (JP)
- KATO, Ikunoshin
Uji-shi, Kyoto 611-0028 (JP)

(74) Representative: VOSSIUS & PARTNER
Siebertstrasse 4
81675 München (DE)

# (54) HOMEOSTASIS-MAINTAINING AGENTS

(57) The present invention provides an agent for maintaining homeostasis in a living body, food, beverage or feed, utilizing a fucoidan, a degradation product thereof or a salt thereof, having an action for maintaining homeostasis in a living body.

The present invention also provides a fucoidan and a marine alga extract with reduced color, reduced bitterness and reduced iodine content, and improved flavor and taste with freshness, a food, beverage, seasoning, feed, cosmetics or medicament, comprising the fucoidan and/or the marine alga extract, and processes for efficiently manufacturing them.

EP 1 327 448 A1

## Description

### TECHNICAL FIELD

The present invention relates to a medicament, food, beverage or feed, having an action for maintaining homeostasis in a living body.

Also, the present invention relates to a fucoidan and a marine alga extract with improved flavor and taste, a food, beverage, seasoning, feed, cosmetics and medicament, comprising the fucoidan and/or the marine alga extract, and a process for manufacturing the same.

### BACKGROUND ART

Marine algae include Phaeophyceae, Chlorophyceae and Rhodophyceae. Among them, Phaeophyceae includes many marine algae which are tasty and healthful, such as *Laminaria, Undaria, Nemacystus* and *Hizikia*, which have been traditionally consumed in a large amount as foods for human. Since these Phaeophyceae do not cause any disorder even when they are consumed in a considerably large amount daily, they are highly safe as foods. Also, Phaeophyceae have been used as a therapeutic agent for cancer in China, and many researchers have tried to identify the substances exhibiting anti-cancer action from the Phaeophyceae. The fucoidan existing in hot water extract from the Phaeophyceae is a kind of a sulfated polysaccharide, containing sulfated fucose. Natural fucoidan has a molecular weight of several hundred thousands or more and has a chemical structure having contradictory characteristics such as highly hydrophilic sulfate group and highly hydrophobic methyl group, so that various components contained in the marine algae tend to adsorb thereto, thereby making its purification very difficult. Recently, as the physiological functions of the fucoidan, there have been elucidated anti-cancer action, action for inducing apoptosis, hypotensive action, anti-allergic action, and the like, thereby making the fucoidan increasingly useful. Therefore, the development of its applications for medicaments and health foods has been expected. Also, since the fucoidan sufficiently exhibits its physiological functions as an effective ingredient of the food, beverage, feed, cosmetics or medicament, there has been desired the development of the process for preparing a high-purity fucoidan conveniently at a high efficiency.

In addition, the marine algae have been generally widely used as food materials and seasoning of soup base. For instance, a root portion of *Laminaria* (*kombu*) was briefly washed with water, and placed in fresh water to extract it for 2 hours or so, and the resulting extract is used for a food as *kombu* water containing *kombu* extract. Alternatively, in order to obtain a *kombu* soup base, the *kombu* is boiled with hot water to prepare a soup base rich in glutamic acid giving a tasty flavor (*umami*). The marine algae contain amino acids, minerals and mucopolysaccharides, and these components are extracted and utilized in food or seasoning. However, the marine algae contain bitter taste components, which are also eluted into a liquid portion by water extraction or hot-water extraction. Also, besides them, there are flavor components and taste components distinctly owned by the marine algae, and coloring components are further increased during extraction by boiling in water. In the extraction of the marine alga, the mucopolysaccharides, minerals, iodine components and odor of the marine alga transfer to the extract even if the extraction is carried out by using either cold water or hot water. Therefore, bitterness and excessive iodine are extracted into the extract, so that it would be difficult to use the extract as a food material for a food or seasoning, in which an odor of the marine alga is not desired. It is important to take mucopolysaccharides and minerals for the health of present-day people, and marine water beverages have been preferred as a drink. However, from the viewpoint of luxury beverage, it is important to reduce the bitterness. Therefore, there have been especially desired the development of a food, beverage and seasoning in which the odor of the marine alga is prevented.

In the removal of the bitterness, the stinginess and excessive coloring components, there have been widely used generally employed ion exchange resin due to chemical reactions and activated carbon treatment due to physical adsorption and such a treatment is effective. However, in these methods, the flavor and taste components inherently owned by the marine alga are exceedingly reduced and the color of the coloring changes. Therefore, the manufactured article has a quality and flavor and taste, far different from that of handmade water extract or boiling water extract of the marine alga. As a result, a real marine alga extract with simple handmade touch cannot be obtained, and a food, beverage, seasoning or feed utilizing such an extract has not been known.

### DISCLOSURE OF INVENTION

The present invention resides in that a new physiological action of the fucoidan is found, and an object thereof is to provide an agent for maintaining homeostasis in a living body, food, beverage or feed, utilizing a fucoidan, a degradation product thereof or a salt thereof.

Another object of the present invention is to provide a fucoidan and a marine alga extract with reduced color, reduced bitterness and reduced iodine content, improved flavor and taste, and freshness, a food, beverage, seasoning,

feed, cosmetics or medicament, comprising the fucoidan and/or the marine alga extract, and processes for efficiently manufacturing them.

Concretely, the gist of the present invention relates to:

[1] an agent for maintaining homeostasis in a living body, characterized in that the agent comprises as an effective ingredient one or more members selected from the group consisting of fucoidan, a degradation product thereof and a salt thereof;

[2] a food, beverage or feed for maintaining homeostasis in a living body, characterized in that the food, beverage or feed comprises one or more members selected from the group consisting of fucoidan, a degradation product thereof and a salt thereof;

[3] a fucoidan obtainable by extracting a marine alga in the presence of a reducing substance;

[4] a marine algae extract obtainable by extracting a marine alga in the presence of a reducing substance;

[5] a food, beverage, seasoning or feed, characterized in that the food, beverage, seasoning or feed comprises the fucoidan of the above [3] and/or the marine alga extract of the above [4];

[6] cosmetics, characterized in that the cosmetics comprise the fucoidan of the above [3] and/or the marine alga extract of the above [4];

[7] a medicament, characterized in that the medicament comprises the fucoidan of the above [3] and/or the marine alga extract of the above [4];

[8] a process for preparing a fucoidan, characterized in that the process comprises the step of extracting a marine alga in the presence of a reducing substance;

[9] a process for preparing a marine alga extract, characterized in that the process comprises the step of extracting a marine alga in the presence of a reducing substance;

[10] a process for manufacturing a food, beverage, seasoning or feed, characterized in that the process comprises the step of the above [8] and/or the step of the above [9];

[11] a process for manufacturing cosmetics, characterized in that the process comprises the step of the above [8] and/or the step of the above [9]; and

[12] a process for manufacturing a medicament, characterized in that the process comprises the step of the above [8] and/or the step of the above [9].

## BRIEF DESCRIPTION OF THE DRAWINGS

Figure 1 is a graph showing an elution pattern of the fucoidan derived from *Kjellmaniella crassifolia* on DEAE-Cellulofine A-800 column.

Figure 2 is a graph showing a suppressive action for elevating a blood sugar level of a rat after eating by the fucoidan.

Figure 3 is a graph showing a suppressive action for elevating a neutral fat level in blood of a rat after eating by the fucoidan.

## BEST MODE FOR CARRYING OUT THE INVENTION

One embodiment of the present invention provides an agent for maintaining homeostasis in a living body, and a food, beverage or feed for maintaining homeostasis in a living body, and one of the major features thereof resides in that each comprises as an effective ingredient one or more members selected from the group consisting of fucoidan, a degradation product thereof and a salt thereof. The exhibition of the desired effects of the present invention is based on the action for maintaining homeostasis in a living body exhibited by the effective ingredient found by the present inventors.

The term "action for maintaining homeostasis in a living body" refers to an action for maintaining a function of life sustenance in a given state for maintaining a living body, and especially refers to an action for maintaining homeostasis in a liver function and an action for maintaining homeostasis in blood. The details of these actions will be described later. Here, "maintaining" is intended to mean regulating so as to keep a given state, in addition to the meaning of keeping at a given state.

The fucoidan which is used as the effective ingredient in the present invention refers to a sulfated fucose-containing polysaccharide, wherein a sulfated fucose is contained as a constituent. The fucoidan is not particularly limited, as long as the fucoidan has the action for maintaining homeostasis in a living body. For instance, algae such as marine algae belonging to Phaeophycae such as Laminariales, Chordariales and Fucales, including *Kjellmaniella crassifolia, Kjellmaniella gyrata, Undaria, Ecklonia kurome, Eisenia bicyclis, Ecklonia cava*, Giant kelp, *Lessonia ni-*

*grescence, Nemacystus, Cladosiphon okamuranus, Fucus* and *Ascophyllum nodosum* especially richly contain fucoidans which have the action for maintaining homeostasis in a living body, and are suitable as raw materials. Also, Echinodermata such as sea cucumber, Echnoidea and Asterozoa may be used as the raw material, and those fucoidans derived from these can be preferably used. Among them, the marine algae of Laminariales richly contain fucoidans which have excellent action for maintaining homeostasis in a living body and are more suitable as the raw materials. Therefore, those derived from algae and those derived from Echinodermata are preferable as the fucoidan which is used as the effective ingredient in the present invention, and those derived from Phaeophycae are more preferable.

These fucoidans may be each prepared by a known method. There can be used in the present invention a crude product, a purified product of a fucoidan, a fucoidan separated into several molecular species, and the like.

For instance, a fucoidan is prepared from *Kjellmaniella crassifolia*, and the resulting fucoidan can be separated into glucuronic acid-containing fucoidan (referred to as "U-fucoidan") and glucuronic acid non-containing fucoidan (referred to as "F-fucoidan"). Each of the fucoidans can be used as the effective ingredient of the present invention. Also, sulfated fucogalactan (hereinafter referred to as "G-fucoidan") can be prepared from *Kjellmaniella crassifolia* and used in the present invention.

After the preparation of the fucoidans from *Kjellmaniella crassifolia*, for instance, U-fucoidan and F-fucoidan are separated by using an anionic exchange resin, a surfactant or the like. The existing ratio of U-fucoidan to F-fucoidan derived from *Kjellmaniella crassifolia* is about 1:2. U-fucoidan contains fucose, mannose, galactose, glucuronic acid and the like, and its sulfate content is about 20%. F-fucoidan contains fucose and galactose, and its sulfate content is about 50%. The molecular weight for both substances is distributed, centering about 200000 (*Summary of 18th Sugar Symposium*, p. 159, 1996).

U-fucoidan and F-fucoidan can be separated, for instance, by applying a fucoidan solution prepared from *Kjellmaniella crassifolia* onto DEAE-Cellulofine A-800 column, and carrying out elution by the concentration gradient technique using NaCl-containing buffer. One of the examples is shown in Figure 1. Concretely, Figure 1 is a diagram showing the separation of U-fucoidan and F-fucoidan, wherein the front peak in the figure is U-fucoidan, and the back peak is F-fucoidan.

In addition, for instance, each of the fucoidan derived from *Nemacystus*, the fucoidan derived from *Cladosiphon okamuranus*, the fucoidan derived from *Undaria*, the fucoidan derived from sporophyll of *Undaria pinnatifida* (Wakame Mekabu) and the fucoidan derived from *Fucus* can be also prepared by a known method, and used in the present invention.

The sea cucumber containing the fucoidan includes, for instance, sea cucumbers described in Japanese Patent Laid-Open No. Hei 4-91027. The fucoidan can be prepared from sea cucumbers in accordance with the method described in the publication. Also, commercially available fucoidans can be used. In addition, the above-mentioned fucoidan can be used by proper sulfation as desired, and the sulfation may be carried out in accordance with the known method.

The degradation product of the fucoidan used as the effective ingredient in the present invention (which may also be hereinafter referred to as "degradation product according to the present invention") can be prepared by degrading the fucoidan with a known method such as an enzymological method, a chemical method, or a physical method, and selecting a desired degradation product using as an index an action for maintaining homeostasis in a living body. In addition, the degradation product of the fucoidan used in the present invention can be used by proper sulfation as desired, and the sulfation may be carried out in accordance with the known method.

The methods for preparing the degradation product of the fucoidan used in the present invention include, for instance, an acid degradation method. By subjecting the fucoidan to an acid degradation, there can be prepared a degradation product having an action for maintaining homeostasis in a living body.

The conditions of the acid degradation for the fucoidan used in the present invention are not particularly limited, as long as the conditions enable to generate the degradation product having an action for maintaining homeostasis in a living body. For instance, the fucoidan is dissolved or suspended in an aqueous solution of an acid or the like and subjected to an acid degradation reaction, thereby generating the degradation product of the present invention. Also, the reaction mixture may be heated during the reaction, thereby shortening the time period required for the generation of the degradation product of the present invention. The kinds of the acid which can be used for acid degradation of the fucoidan are not particularly limited. There can be used an inorganic acid such as hydrochloric acid, sulfuric acid or nitric acid; an organic acid such as citric acid, formic acid, acetic acid, lactic acid or ascorbic acid; and a solid acid such as cationic exchange resin, cationic exchange fiber or cationic exchange membrane.

The concentration of the acid is not particularly limited, and the acid can be used at a concentration of preferably from 0.0001 to 5 N or so, more preferably from 0.01 to 1 N or so. In addition, the reaction temperature is not particularly limited, and the reaction temperature may be set at preferably from 0° to 200°C, more preferably from 20° to 130°C.

In addition, the reaction time is not particularly limited, and the reaction time may be set at preferably from several seconds to several days. The kinds and the concentration of the acids, the reaction temperature, and the

reaction time may be properly selected depending upon the generated amount of the degradation product according to the present invention and the degree of polymerization of the degradation product. For instance, in the preparation of the degradation product of the present invention, by properly selecting an organic acid such as citric acid, lactic acid or malic acid at a concentration of the acid from the range of several dozens mM to several M, a heating temperature in the range of from 50° to 110°C, preferably from 70° to 95°C, and a heating time in the range of from several minutes to 24 hours, the acid degradation product of the fucoidan can be prepared. The degradation product is exemplified by the acid degradation product of the fucoidan derived from *Kjellmaniella crassifolia,* and the degradation product can be used as a dietary fiber having an action for maintaining homeostasis in a living body.

The degradation product of the present invention can be fractionated by using as an index its action for maintaining homeostasis in a living body. For instance, the acid degradation product can be subjected to a molecular weight fractionation by means of a gel filtration method, a fractionation method using a molecular weight fractionation membrane, or the like.

As an example of the gel filtration method, Cellulofine GCL-300 can be used to prepare any molecular weight fractions, for instance, one having a molecular weight exceeding 25000, one having a molecular weight of 25000 to exceeding 10000, one having a molecular weight of 10000 to exceeding 5000, one having a molecular weight of 5000 or less, and the like. Cellulofine GCL-25 can be used to prepare any molecular weight fractions from the fraction having a molecular weight of 5000 or less, for instance, one having a molecular weight of 5000 to exceeding 3000, one having a molecular weight of 3000 to exceeding 2000, one having a molecular weight of 2000 to exceeding 1000, one having a molecular weight of 1000 to exceeding 500, one having a molecular weight of 500 or less and the like.

In addition, the degradation product of the present invention can be subjected to the molecular weight fractionation on an industrial scale using an ultrafiltration membrane. For instance, a fraction having a molecular weight of 30000 or less can be prepared by using FE10-FUSO382 manufactured by DAICEL CHEMICAL INDUSTRIES, LTD., or a fraction having a molecular weight of 6000 or less can be prepared by using FE-FUS-T653 manufactured by the same. Further, a fraction having a molecular weight of 500 or less can be obtained by using a nanofilter membrane. Any molecular weight fractions can be prepared by combining these gel filtration methods and molecular weight fractionation methods.

The degradation product of the fucoidan having an action for maintaining homeostasis in a living body, which can be used in the present invention, is exemplified by the compound represented by the formulas (I) to (IV), and these compounds can be prepared in accordance with the methods disclosed in WO 97/26896, WO 99/41288, and WO 00/50464. Also, the degradation product according to the present invention is exemplified by the degradation product of the fucoidan described in WO 97/26896, WO 99/41288 and WO 00/50464.

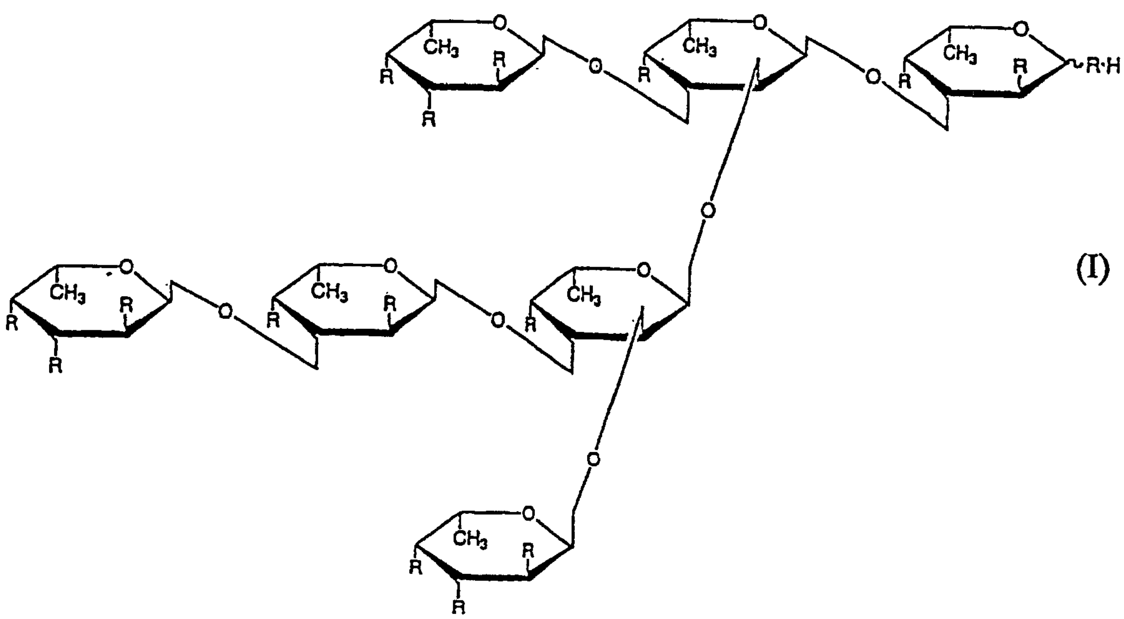

(I)

wherein R is OH or $OSO_3H$;

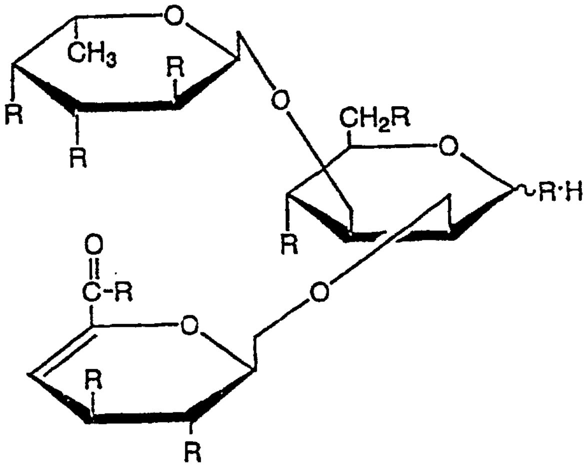

(II)

wherein R is OH or $OSO_3H$;

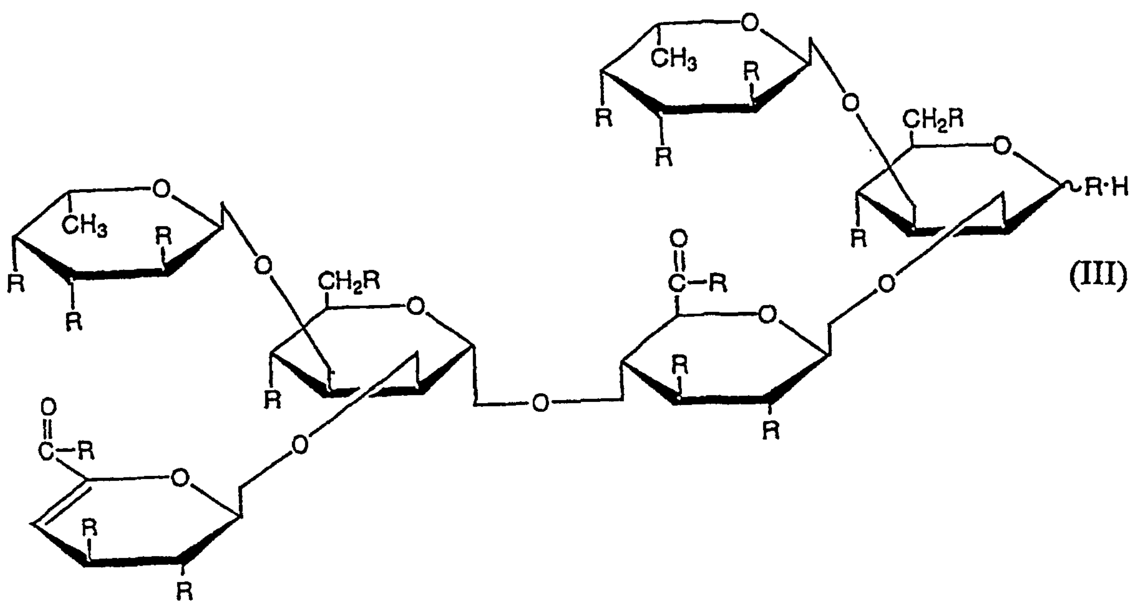

(III)

wherein R is OH or $OSO_3H$; and

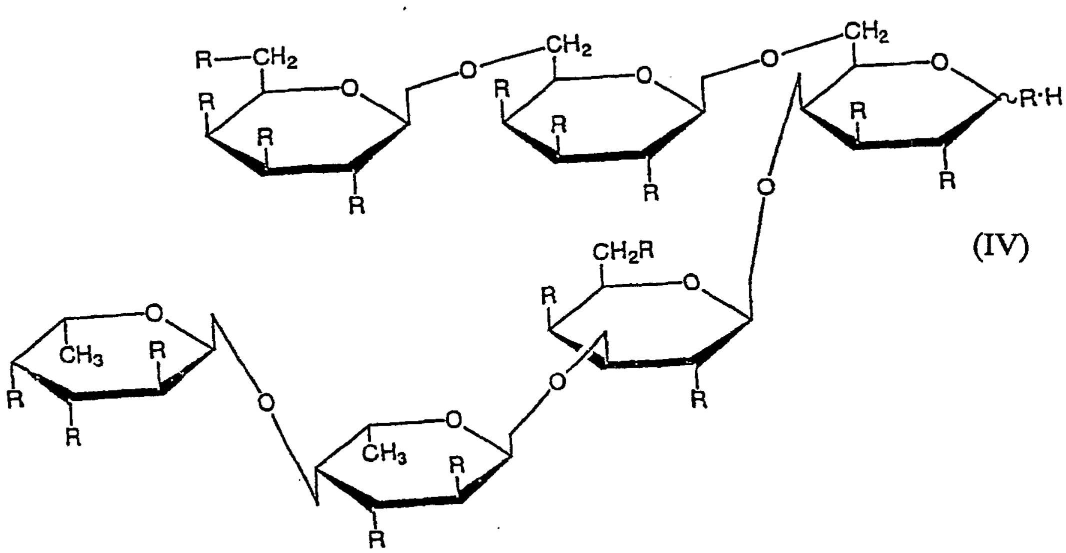

(IV)

wherein R is OH or $OSO_3H$.

Examples of the compound represented by the formula (I) include the compound represented by the formula (V) given later. Examples of the compound represented by the formula (II) include the compound represented by the formula (VI) and the formula (VII) given later. Examples of the compound represented by the formula (III) include the compound represented by the formula (VIII).

The compound represented by the formula (I) can be obtained by, for instance, degrading the above-mentioned F-fucoidan with endo-sulfated polysaccharide degrading enzyme (F-fucoidan-specific degradation enzyme) produced by *Alteromonas* sp. SN-1009 (FERM BP-5747), and purifying the resulting degradation product. As to the content and the site of sulfate group in the compound, any ones can be purified from the degradation products. In addition, the polymer of the compound represented by the formula (I) is contained in the degradation products, and can be separated and purified depending on its purposes.

Each of the compounds represented by the formula (II) and the formula (III) can be obtained by, for instance, degrading the above-mentioned U-fucoidan with endo-sulfated polysaccharide degrading enzyme (U-fucoidan-specific degradation enzyme) produced by *Flavobacterium* sp. SA-0082 (FERM BP-5402), and purifying the resulting degradation products. As to the content and the site of sulfate group in the compound, any ones can be purified from the degradation products. In addition, the polymer of which basic backbone structure comprises the compound represented by the formula (II) or the formula (III) is also contained in the degradation products, and can be separated and purified depending on its purposes.

The above-mentioned G-fucoidan can be prepared by degrading the fucoidan derived from *Kjellmaniella crassifolia* with F-fucoidan-specific degradation enzyme produced by *Alteromonas* sp. SN-1009 (FERM BP-5747) and U-fucoidan-specific degradation enzyme produced by *Flavobacterium* sp. SA-0082 (FERM BP-5402), and purifying the resulting degradation product.

The above-mentioned *Flavobacterium* sp. SA-0082 (PERM BP-5402) also produces endo-sulfated polysaccharide degrading enzyme, which specifically degrades G-fucoidan (G-fucoidan-specific degradation enzyme). The G-fucoidan is treated with the G-fucoidan-specific degradation enzyme to prepare a degradation product of the G-fucoidan, and the degradation product is purified according to its purpose, whereby a degradation product which can be used as an effective ingredient according to the present invention can be prepared from the above degradation product. The compound represented by the formula (IV) is one such example. As to the content and the site of sulfate group in the compound, any ones can be purified from the degradation products. In addition, the polymer of which basic backbone structure comprises the compound represented by the formula (IV) is also contained in the above degradation products, and can be separated and purified according to its purposes.

Each of the above-mentioned enzymes is described in WO 97/26896 and WO 00/50464. Also, each of the above-mentioned F-fucoidan-specific degradation enzyme, U-fucoidan-specific degradation enzyme and G-fucoidan-specific degradation enzyme can be prepared from a recombinant with an isolated gene of the enzyme obtained by a known method, and used. For instance, the F-fucoidan-specific degradation enzyme and the U-fucoidan specific degradation enzyme can be each prepared from a recombinant according to the method described in WO 99/11797, and used.

Specified fractionated products of the above-mentioned fucoidan, especially F-fucoidan, U-fucoidan and G-fucoidan, are fucoidan-fractionated products of which basic structure have been made definite for the first time. Each degradation product prepared by using an enzyme capable of specifically acting to each fractionated product, especially a fractionated product of the degradation product thereof such as the above-mentioned compounds of the formula (I) to formula (IV) have remarkably more excellent characteristics from the viewpoints of having a molecular weight smaller than that of the fucoidan and having physiological activities of the same level as that of the fucoidan, as compared to the simply separated fucoidan of which structure, composition and properties are indefinite, or a fucoidan-containing product.

The salt of the fucoidan or a degradation product thereof used as the effective ingredient in the present invention is exemplified by, for instance, alkali metal salts, alkaline earth metal salts, salts with organic bases and the like. Examples thereof include salts with sodium, potassium, calcium, magnesium, ammonium, diethanolamine, ethylenediamine, and the like. These salts are obtained by, for instance, converting sulfate group or carboxyl group existing in fucoidans and the like to salts according to a known method. The salt is preferably a pharmacologically acceptable salt.

Furthermore, in the first embodiment of the present invention, there can be preferably used as an effective ingredient a fucoidan, a degradation product thereof and a salt thereof, each having an action for maintaining homeostasis in a living body, which can be obtained according to the process for preparing a marine alga extract of the present invention, which will be described in detail in the explanation of the second embodiment of the present invention mentioned later. The fucoidan can be obtained by a process for preparing a fucoidan in a high purity and high yield, comprising extracting a desired raw material in the presence of a reducing substance. The resulting fucoidan has the characteristics of a lighter color in external appearance and more reduced odor of a marine alga, as compared to the fucoidan obtained by a conventional process for preparing a fucoidan, so that the fucoidan obtained is more preferred as the effective ingredient in the embodiment of the present invention. The process for preparing a fucoidan is the same as the process for preparing a marine alga extract mentioned later for the conditions of treating raw materials, the conditions of extracting the desired substance from the raw materials and the like, except that the above-mentioned marine algae (preferably Phaeophyceae) as the raw materials, which have been known to contain the fucoidan or thought to contain the fucoidan are used. The fucoidan (which may be referred to as "fucoidan extracted under reduction") and a process for preparing the fucoidan are encompassed in the present invention.

The exhibition of the action for maintaining homeostasis in a living body of the effective ingredient of the present invention can be evaluated by the methods described in Examples set forth below. For instance, the action for maintaining homeostasis in a liver function can be evaluated by the methods described in Examples 1 to 4. In addition, the action for maintaining homeostasis in blood can be evaluated by the methods described in Examples 5 and 6.

The agent for maintaining homeostasis in a living body as a medicament in the first embodiment of the present invention encompasses all medicaments exhibiting pharmacological effects based on the actions of the effective ingredient, such as a medicament for preventing or treating a disease or pathology due to change in the homeostasis (or a disease or pathology requiring maintenance of homeostasis in a living body) in a living body by an action for maintaining homeostasis in a living body of the above-mentioned effective ingredient. Among them, the effective ingredient has excellent actions for maintaining homeostasis in a liver function and in blood. In a preferred embodiment of the present invention, there is provided an agent for maintaining homeostasis in a living body as a therapeutic agent or prophylactic agent for a liver function disorder or as an agent for maintaining homeostasis in blood.

The action for maintaining homeostasis in a liver function owned by the effective ingredient of the present invention refers concretely to an action for treating or preventing a liver function disorder. Therefore, the agent for maintaining homeostasis of the present invention is effective for treatment or prevention of a disease or pathology caused by change in the homeostasis in a liver function, especially a liver function disorder.

The liver function disorders to be treated or prevented by the agent for maintaining homeostasis of the present invention used as the therapeutic agent or prophylactic agent for liver function disorders (hereinafter referred to as "therapeutic agent or prophylactic agent of the liver function disorders of the present invention") are not particularly limited. The liver function disorders include, for instance, severe hepatitis, fulminant hepatitis, alcoholic hepatitis, chronic hepatitis, cirrhosis, liver cancer, and the like, preferably chronic hepatitis, alcoholic hepatitis or cirrhosis which is a disease accompanying hepatic fibrosis. Furthermore, the liver cancer which is onset via these disorders is also encompassed as a disease accompanying hepatic fibrosis.

The hepatic fibrosis is a pathology in which binding tissues such as collagen are increased and accumulated in the liver as found in chronic hepatitis, alcoholic hepatitis and cirrhosis. Therefore, the above-mentioned diseases can be treated or prevented by suppressing the development of hepatitis fibrosis. On the other hand, when the chronic hepatitis or cirrhosis is worsened, the risk that the disease would progress to a liver cancer becomes high. From this finding, the suppression of hepatic fibrosis is extremely useful in the treatment and prevention of a liver cancer.

In addition, these causative factors for liver function disorders include, but not particularly limited to, causative factors such as viruses, autoimmune, drugs, alcohols, nutritional disorders, inborn errors of metabolism and circulatory disorders, and the agent for maintaining homeostasis of the present invention can be used for liver function disorders

due to any of these causations. Also, the effective ingredient of the present invention is especially useful for the alcoholic liver function disorders as shown in Examples 3 and 4 set forth below.

On the other hand, the action for maintaining homeostasis in blood owned by the effective ingredient of the present invention refers to an action for suppressing the elevation of the blood sugar level and the neutral fat level in blood which are causatives of a lifestyle-related disease (especially diabetes, obesity, or hyperlipemia), an action for lowering the blood sugar level and the neutral fat level elevated in blood, and the like, and namely an action for suppressing the elevation of the blood sugar level, an action for suppressing the elevation of the neutral fat level in blood, and the like. Here, the term "suppressing" is intended to include regulating. The effective ingredient of the present invention is especially excellent in the above-mentioned action concretely described as the action for maintaining homeostasis in blood. Therefore, according to the present invention, there is provided an agent for maintaining homeostasis in a living body having these actions. According to the agent for maintaining homeostasis, the elevation in the blood sugar level or the neutral fat level in blood is suppressed or the elevated blood sugar level or neutral fat level in blood is lowered, whereby the agent can be used for the prevention or treatment of the life-style diseases, especially diabetes, obesity and hyperlipemia. Also, the effective ingredient of the present invention has an action for suppressing the elevation in the blood sugar level and the neutral fat level in blood after eating or drinking. Therefore, according to the present invention, there is provided an agent for maintaining homeostasis in a living body as the agent for maintaining homeostasis in blood after eating or drinking. The agent for maintaining homeostasis in blood is very useful for the treatment or prevention of patients with diabetes, obesity and hyperlipemia of which diet is under restriction. The neutral fat as referred herein is exemplified by monoglyceride, diglyceride, triglyceride, and the like.

Next, the method for preparing an agent for maintaining homeostasis of the present invention will be explained. The agent for maintaining homeostasis in a living body of the present invention comprises as an effective ingredient one or more members selected from the group consisting of fucoidan, a degradation product thereof and a salt thereof, and may be combined with a known medicinal vehicle to make a preparation. The preparation is generally produced by formulating the effective ingredient according to the present invention with a pharmacologically acceptable liquid or solid vehicle, and optionally adding a solvent, a dispersant, an emulsifier, a buffer, a stabilizer, an excipient, a binder, a disintegrant, a lubricant, or the like, and forming the resulting mixture into a solid agent such as a tablet, a granule, a powder, a fine powder, or a capsule, or a liquid agent such as a general liquid agent, a suspension agent, or an emulsion agent. In addition, the medicament can be formed into a dry product which can be made into a liquid form by adding an appropriate vehicle immediately before use, or into external preparation.

The medicinal vehicle can be selected depending upon the above-mentioned administration form and preparation form of the agent for maintaining homeostasis of the present invention. In the case of an orally administered preparation, there can be utilized, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated.

On the other hand, a non-orally administered preparation can be prepared by dissolving or suspending the effective ingredient according to the present invention, in a diluent such as distilled water for injection, physiological saline, an aqueous solution of glucose, vegetable oil for injection, sesame oil, peanut oil, soybean oil, corn oil, propylene glycol or polyethylene glycol, according to a conventional method, and adding optionally a microbicide, a stabilizer, an osmotic regulator, a soothing agent, or the like.

External preparation includes solid, hemi-solid or liquid preparation for percutaneous administration. The external preparation also includes suppositories and the like. For instance, the external preparation may be prepared as liquid preparations including emulsions, suspensions such as lotions and external tinctures; ointments including oily ointments and hydrophilic ointments; medical adhesives for percutaneous administration such as films, tapes and poultices; and the like.

The agent for maintaining homeostasis can be appropriately manufactured by known methods in the field of pharmaceuticals. The content of the effective ingredient according to the present invention in the agent for maintaining homeostasis is not particularly limited, as long as the content is preferably the amount so that the effective ingredient can be administered within the dose range described later in consideration of administration form, administration method and the like of the preparation.

The agent for maintaining homeostasis in a living body of the present invention can be administered via an administration route appropriate for each of the preparation form. The administration method is not limited to specific one. The agent can be administered internally, externally (or topically) and by injection. The injection can be administered, for instance, intravenously, intramuscularly, subcutaneously, percutaneously, or the like. The external preparation includes suppositories and the like.

The dose of the agent for maintaining homeostasis in a living body of the present invention is changeable and properly set depending upon its preparation form, administration method, purpose of use, and age, body weight, or symptom of a patient to whom the agent is applied, and the like. Generally, the dose of the agent in terms of the

above-mentioned effective ingredient contained therein is preferably from 0.1 to 2000 mg/kg per day for adult. As a matter of course, the dose varies depending upon various conditions, so that an amount smaller than the dose mentioned above may be sufficient, or an amount exceeding the dose range may be required. Also, the agent for maintaining homeostasis of the present invention can not only be orally administered *per se* but also be taken on a daily basis by adding the agent to optional foodstuff. In addition, the fucoidan, a degradation product thereof and/or a salt thereof may be used as raw materials for foodstuff for maintaining homeostasis in a living body.

In addition, due to its action for maintaining homeostasis in a living body, the fucoidan, a degradation product thereof and/or a salt thereof which is used as an effective ingredient according to the present invention is useful for those pertaining to the maintenance of homeostasis in a living body, for instance, studies on onset of liver function disorders, studies on mechanisms of suppression of the elevation of the blood sugar level or the elevation of the neutral fat level in blood, and screening of an agent for ameliorating liver functions, an agent for suppressing the elevation of the blood sugar level and an agent for suppressing the elevation for the neutral fat level in blood.

In addition, as another embodiment of the present invention, there is provided a method for maintaining homeostasis in a living body, comprising administering the above-mentioned effective ingredient to an individual. The method is especially useful for treating or preventing a liver function disorder, or maintaining homeostasis in blood. The fucoidan to be administered to an individual is preferably those derived from algae or those derived from Echinodermata, and those belonging to Phaeophycae are preferable as the algae. Also, it is especially preferable to administer the above-mentioned fucoidan extracted under reduction. Here, an individual refers to a mammal, especially human.

This method can be carried out by administering the above-mentioned effective ingredient, preferably the agent for maintaining homeostasis of the present invention to human who is expected to need, for instance, the maintenance of homeostasis in a living body, or who requires such maintenance. The method for administration, the dose and the like of the effective ingredient may be in accordance with the method for administration, the dose or the like of the agent for maintaining homeostasis of the present invention used in administration of the effective ingredient. Here, in this embodiment, the food, beverage or feed mentioned later can be also used.

Next, the food, beverage or feed of the present invention comprising the effective ingredient according to the present invention (which may be hereinafter referred to as "food, beverage or feed for maintaining homeostasis in a living body of the present invention") will be explained. Due to the action for maintaining homeostasis in a living body owned by the effective ingredient, the food, beverage or feed of the present invention is effective for maintaining homeostasis in a living body showing sensitivity to the effective ingredient, and thus being very useful for amelioration or prevention of the diseases requiring the maintenance of homeostasis. According to the present invention, there is provided a food, beverage or feed which has the same actions and effects as the above-mentioned agent for maintaining homeostasis. The preferred embodiments are the same as the above-mentioned agent for maintaining homeostasis.

Here, the term "comprising" in connection with the food, beverage or feed of the present invention encompasses "containing," "adding" and "diluting. The term "containing" refers to an embodiment of containing the effective ingredient used in the present invention in the food, beverage or feed; the term "adding" refers to an embodiment of adding the effective ingredient used in the present invention to a raw material for the food, beverage or feed; and the term "diluting" refers to an embodiment of diluting the effective ingredient used in the present invention with a raw material for the food, beverage or feed.

The process for preparing the food or beverage for maintaining homeostasis in a living body according to the present invention is not particularly limited. The method includes cooking, processing, and any process by which foods or beverages can be generally prepared, as long as the fucoidan, a degradation product thereof and/or a salt thereof, having an action for maintaining homeostasis in a living body, is contained, added and/or diluted as the effective ingredient in the food or beverage prepared.

The food or beverage for maintaining homeostasis in a living body according to the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, and rice snacks; alcohol beverages such as *sake,* Chinese liquor, wine, whisky, Japanese distilled liquor (*shochu*), vodka, brandy, gin, ram, beer, refreshing alcoholic beverages, fruit liquor, and liqueur; luxury drinks such as green tea, tea, oolong tea, coffee, refreshing beverages

and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like.

The content of the effective ingredient according to the present invention in the food or beverage of the present invention is not particularly limited, and the content can be appropriately selected from the viewpoints of sensory ability and exhibition of the actions. The content of the effective ingredient is, for instance, preferably 0.0001 parts by weight or more, more preferably from 0.001 to 10 parts by weight, per 100 parts by weight of the food, or for instance, preferably 0.0001 parts by weight or more, more preferably from 0.001 to 10 parts by weight, per 100 parts by weight of the beverage.

In addition, according to the present invention, there is provided a feed for an organism prepared by containing, adding and/or diluting the effective ingredient according to the present invention. In still another embodiment, the present invention also provides a method of feeding an organism, characterized by administering the above-mentioned effective ingredient to the organism. In still yet another embodiment, the present invention provides an organism feeding agent characterized in that the organism feeding agent comprises the above-mentioned effective ingredient.

In these inventions, the organism includes, for instance, culturing or breeding animals, pet animals, and the like. The culturing or breeding animal is exemplified by cattle, laboratory animals, poultry, pisces, crustaceae or shellfish. The feed is exemplified by a feed for sustenance of and/or improvement in physical conditioning. The organism feeding agent is exemplified by immersion agents, feed additives, and beverage additives.

According to these inventions, there can be expected to exhibit the same effects as those of the above-mentioned agent for maintaining homeostasis of the present invention in the organism exemplified above for applying these, on the basis of the action for maintaining homeostasis in a living body owned by the effective ingredient according to the present invention.

In the feed according to the present invention, the above-mentioned effective ingredient used in the present invention is usually administered in an amount of preferably from 0.01 to 2000 mg per 1 kg of the subject organism per day. The administration can be made using a feed prepared by adding and mixing the effective ingredient in a raw material for an artificially formulated feed, or by mixing the effective ingredient with a powder raw material for an artificially formulated feed, and thereafter further adding and mixing the resulting mixture with other raw materials. The content of the effective ingredient according to the present invention in the feed for a subject organism is not particularly limited. The content can be appropriately set in accordance with its purposes, and an appropriate proportion in the feed is preferably in a ratio of from 0.001 to 15% by weight.

The artificially formulated feed includes feeds using animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and vitamins, minerals, amino acids, and antioxidants; and the like as raw materials. In addition, feeds for fish such as fish minced meat are also included.

The process for preparing the feed according to the present invention is not particularly limited. The feed can be prepared according to a general method for a feed, as long as the effective amount of the above-mentioned effective ingredient according to the present invention may be contained, added and/or diluted in the feed produced.

Also, the effective ingredient according to the present invention can be administered by directly adding the effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the above-mentioned effective ingredient used in the present invention in water or seawater is not particularly limited, and the concentration may be set in accordance with its purposes. It is appropriate that the concentration is preferably from 0.00001 to 1% by weight.

Also, a beverage comprising the effective ingredient according to the present invention may be given to a subject organism as a feeding drink. The concentration of the effective ingredient according to the present invention in the beverage is not particularly limited, and the concentration may be set in accordance with its purposes. It is appropriate that the concentration is preferably from 0.0001 to 1% by weight. The organism feeding agent *per se,* for instance, an immersion agent, a feed additive, or a beverage additive comprising the above-mentioned effective ingredient according to the present invention may be prepared by a known method. The content of the effective ingredient in the organism feeding agent is not particularly limited, so long as the desired effects of the present invention can be obtained.

The organism to which these feeds or the like according to the present invention can be applied is not limited.

The culturing or breeding animals include cattle such as *Equus, Bos, Porcus, Ovis, Copra, Camelus*, and *Lama;* laboratory animals such as mice, rats, guinea pigs, and rabbits; poultry such as *Chrysolophus*, ducks, *Meleagris*, and *Struthionifcrmes;* pisces such as *Pagrus, Oplegnathidae, Paralichthys*, plaice, *Seriola*, young *Seriola*, amberjack, *Thunna, Caranx delicatissimus, Plecoglossus, Salmo •Oncorhynchus, Fugu, Anguilla, Misguirus,* and *Parasilurus*; Crustaceae such as *Penaidae*, black tiger shrimp, *Penaeus roentalis*, and *Portulus trituberculatus*; and shellfish such as abalones (*awabi*), turban shells, scallops, and oysters; and the pet animals include dogs, cats, and the like, so that the feed can be widely applied to animals on land and in water.

The process for feeding an organism provided by the present invention, in which the effective ingredient according to the present invention is administered to the organism, can be carried out, for instance, by administering the above-mentioned feed and/or organism feeding agent according to the present invention to an organism to be administered so that the desired effects of the present invention can be obtained.

By allowing a subject organism to take the feed according to the present invention, or immersing a subject organism into a solution containing the above-mentioned effective ingredient according to the present invention, the physical conditioning of the cattle, laboratory animals, poultry, pisces, Crustacea, shellfish, pet animals or the like can be well sustained and improved.

The food, beverage or feed for maintaining homeostasis in a living body of the present invention does not have any particular limitation on its shape, as long as the fucoidan, a degradation product thereof and/or a salt thereof, which has an action for maintaining homeostasis in a living body, used as the effective ingredient is contained therein, added thereto and/or diluted therewith, and the feed, beverage or feed comprises the effective ingredient in an amount required for exhibiting its action. Such shapes include orally taken shapes such as tablets, granules and capsules. Here, the effective ingredient according to the present invention is health food material having both the action for maintaining homeostasis in a living body and dietary fiber function, so that the effective ingredient is extremely useful as a material for manufacturing a food, beverage or feed.

A still another embodiment of the present invention provides use of one or more members selected from the group consisting of a fucoidan, a degradation product thereof and a salt thereof, used for manufacturing a medicament for maintaining homeostasis in a living body.

A second embodiment of the present invention provides a fucoidan and a marine alga extract, having reduced color, reduced bitterness and iodine content, with freshness, improved taste and flavor; a food, beverage, seasoning, feed, cosmetics or medicament, comprising the fucoidan and/or marine alga extract; and processes for efficiently manufacturing these. In the embodiment of the present invention, the fucoidan and the marine alga extract usable for the food, beverage, seasoning, feed, cosmetics or medicament can be obtained according to the process for preparing a fucoidan or marine alga extract, provided by the present invention. The fucoidan and the marine alga extract are those in which ingredients which cause odor of a marine alga, pigments, proteins and the like are reduced or removed, thereby respectively preferably providing as a fucoidan and a marine alga extract which is odorless or has reduced odor of a marine alga, or a fucoidan and a marine alga extract which is colorless or has reduced green-brown color derived from a marine alga. In the case where the fucoidan or marine alga extract is mixed and used with other components as the raw materials, there would be no influences in flavor or taste when used in a high concentration, so that the characteristics of the other components can be fully exhibited. Also, as described later, when the protease is used in the process for preparing a fucoidan or marine alga extract of the present invention, the protein which can be an allergen has been markedly reduced or removed in the fucoidan or marine alga extract obtained, so that it is especially useful as materials for the food, beverage, seasoning, feed, cosmetics or medicament in which allergic reactions are to be avoided. Furthermore, since the fucoidan or marine alga extract of the present invention has a reduced iodine content, and excessive intake of iodine is said to be not good for the body, the fucoidan or marine alga extract is useful as materials for the food, beverage, seasoning, feed, cosmetics or medicament. Also, the fucoidan is degraded in the same manner as in the first embodiment of the present invention, and can be used in the same manner in the form of the degradation product of the fucoidan as the above fucoidan.

Since the fucoidan and marine alga extract of the present invention have little impurity, the removal of the protein or the removal of the by-products by the impurities can be reduced or omitted during its purification process even when the fucoidan or marine alga extract is re-processed, or enzymatically and/or chemically reacted with other ingredients, so that the fucoidan and marine alga extract can be excellently used as the precursor substance. For instance, the fucoidan and marine alga extract are preferably used in the formation of oligosaccharides due to hydrolysis by an enzyme or acid, and chemical synthesis. Furthermore, since the impurities as the raw material is small, the chemical changes between the ingredients are less likely to be caused during storage, so that the quality of the ingredients can be stabilized.

The color, the protein content and the iodine content of the fucoidan or marine alga extract of the present invention can be determined as concretely described in Example 7 or 14 set forth below. As to the color, those having absorbance at a wavelength of 660 nm of 0.001 to 0.03 are preferable, more preferably from 0.01 to 0.02. The protein content is preferably from 0.01 to 8 g/100 g, more preferably from 0.1 to 7 g/100 g. The iodine content is preferably

from 0.1 to 5.5 mg% (w/v), more preferably from 0.2 to 5 mg% (w/v).

The fucoidan provided in the embodiment of the present invention is provided as a compound *per se*, or as a fucoidan fraction, specifically a composition of a fucoidan and other ingredients partly contained therein. Also, the amounts of the fucoidan, the marine alga extract and their raw materials are referred to their weights on dry basis unless specified otherwise.

Furthermore, the fucoidan and marine alga extract of the present invention have various physiological actions known therefor, which can exhibit, for instance, an inducing action for apoptosis, an enhancing action for hepatocyte growth factor, a regulatory action for cytokine production, hair restoring action, an action for lowering cholesterol, an action for clarification of blood, an action for anti-coagulation, an action for anti-cancer, an action for anti-AIDS virus, an action for anti-tumor and the like. Especially, the fucoidan and marine alga extract are excellent in their action for lowering cholesterol, action for clarification of blood, action for anti-coagulation, action for anti-cancer, action for anti-AIDS virus and action for anti-tumor.

Therefore, the food, beverage, seasoning, feed, cosmetics or medicament provided by the present invention (which may hereinafter be referred to as "food or the like of the present invention"), comprising the fucoidan and/or marine alga extract of the present invention described above has excellent applicability to human from the various aspects. On the bases of various actions for the fucoidan and/or marine alga extract, especially an action for lowering cholesterol, an action for clarification of blood, an action for anti-coagulation, an action for anti-cancer, an action for anti-AIDS virus and an action for anti-tumor, according to the medicament, for instance, of the present invention, effects for ameliorating or mitigating diseases showing sensitivity to the above-mentioned actions, and effects for treating or preventing the diseases can be expected. Therefore, as the preferred medicament of the present invention, there is provided a medicament for lowering cholesterol, a medicament for clarification of blood, a medicament for anti-coagulation, a medicament for anti-cancer, a medicament for anti-AIDS virus, or a medicament for anti-ulcer.

In the food or the like of the present invention, the above-mentioned "fucoidan extracted under reduction" is used. Also, the fucoidan obtainable from the marine alga extract provided by the present invention can be used. On the other hand, the marine alga extract usable for the food or the like of the present invention can be obtained by the method concretely shown as follows. The marine alga extract and the method for preparing the marine alga extract are encompassed in the present invention.

One of the major features of the process for preparing a marine alga extract resides in that the process comprises the step of extracting a marine alga in the presence of a reducing substance.

The marine algae to be used as the raw materials may be any one as long as they are algae, including, for instance, those belonging to Phaephyceae (Phaeophyta) such as Fucales, Dictyotales, Laminariales, Sporochnales, Sphacelariales, Cutleriales, Dictyosiphonales, Scytosiphonales, Chordiariales, Ralfsiales, and Ectocarpales; those belonging to Rhodophyceae (Rhodophyta) such as Ceramiales, Rhodymeniales, Nemaliales, Gigartinales, Cryptonemiales, Rhodymeniales and Gelidiales; those belonging to Chlorophyceae (Chlorophyta) such as Codiales, Dasycladales, Ulvales and Caulerpales; and the like. Especially, Phaephyceae are preferable. When the Phaephyceae are used in the present invention, a marine alga extract comprising a fucoidan as its functional ingredient can be obtained, and a fucoidan extracted under reduction can be obtained from the above marine alga extract in high purity and high yield. Among them, those of Laminariaceae and Alariaceae, belonging to Laminariales, are especially preferable. As those of Laminariaceae, there can be used, for instance, *Laminaria japonica, Laminaria japonica var. ochotensis okamura, Laminaria religiosa, Laminaria angustata, Kjellmaniella crassifolia,* and the like. In addition, as those of Alariaceae, there can be used, for instance, *Undaria, Undaria undaricides, Undaria peterseniana* and the like. Further, *Undaria* can be used as either or both of thallus and sporophyll thereof.

In the process for preparing a marine alga extract of the present invention, the process for the pretreatment of the raw materials before the extraction is not particularly limited, and simple water-washing, heat-blasting with dry heated air, roasting, or steaming may be carried out in accordance with an ordinary method. Alternatively, a protease treatment may be carried out as described later, or a combination of these treatments may be carried out. The shape of the marine algae after the pretreatment is not particularly limited, and the marine alga may be used in the form of powders, flakes, thinly cut slices, square-cut pieces or thin pieces, or as it is.

The reducing substance used in the present invention is not particularly limited, and those which can be used for food are preferable. It is preferable to use one or more members selected from the group consisting of ascorbic acid, ascorbates, erythorbic acid, erythorbates, cysteine and glutathione, from the viewpoints of the reducing effects and the influences on flavor. Here, the ascorbate and the erythorbates are preferably salts with sodium, potassium, calcium and the like. The amount of the reducing substance used is not particularly limited. The amount of the reducing substance is preferably from 0.005 to 1.0 part by weight, more preferably from 0.01 to 0.1 parts by weight, based on 100 parts by weight of the solvent used for the extraction.

In addition, it is preferable that the marine alga extract is extracted from a marine alga in the presence of a reducing substance using hot water or a solvent, from the viewpoint of production efficiency. Further, it is more preferable that the marine alga is previously subjected to protease treatment and thereafter extracted in the presence of a reducing

substance using hot water or a solvent, or the marine alga is extracted in the presence of a reducing substance using hot water or a solvent, with subjecting to protease treatment. Here, it is preferable that the temperature of the hot water is within the extraction temperature described later.

The extraction method is not particularly limited. Known hot water immersion method, hot water spraying method, or hot water circulation method is preferred, from the viewpoint of operability. Besides them, the extraction can be carried out by supercritical extraction method.

The extraction conditions in the present invention are not particularly limited. For instance, the extraction can be carried out in accordance with known conditions in the above-mentioned extraction methods.

In a preferred embodiment, the weight of the marine alga used as a raw material (on a dry basis) is preferably 0.5 parts by weight or more and less than 25 parts by weight, based on 100 parts by weight of a total of the solvent and the dry marine alga used in the extraction, from the viewpoint of production efficiency. From the viewpoints of flavor and taste, the weight of the marine alga is more preferably from 1 to 10 parts by weight. Here, the raw material can be extracted in a rate of 0.5 to 25 parts by weight, and thereafter the resulting marine alga extract may be concentrated to increase the concentration of the ingredients and used, or the marine alga extract may be powdered. Further, the marine alga extract may be diluted. The solvent used in the extraction is not particularly limited, as long as the solvent is non-toxic. A drinkable one can be used as the solvent. For instance, water or a solvent can be properly used. As the solvent, organic and inorganic solvents can be used, including, for instance, ethyl alcohol, an aqueous solution of ethyl alcohol, and the like. Also, when the supercritical extraction is carried out, there is included carbon dioxide gas and the like. Water can be preferably used as the solvent, from the viewpoint of easy handling. Water used in the extraction is not particularly limited, and any of those which are drinkable are preferable. Among them, desalted water and distilled water are preferable, and tap water can also be used.

The extraction temperature is preferably from 30° to 130°C, more preferably from 75° to 130°C, especially preferably from 50° to 100°C, from the viewpoint of extraction efficiency. Also, the extraction temperature is more preferably from 80° to 100°C, from the viewpoint of the sensory qualities of the marine alga extract. The extraction time is preferably from 5 minutes to 32 hours, more preferably from 0.2 to 24 hours, still more preferably from 0.5 to 5 hours, from the viewpoint of production efficiency. The pH of the extract during the extraction is preferably from 3 to 7. Also, it is preferable that the pH after the extraction is from 3 to 6 from the viewpoints of storage and retention of flavor and taste. The process for solid-liquid separation of the extract containing the extracted product from the marine alga from the extraction residue is not particularly limited. The filtration or centrifugation may be carried out by an ordinary process; alternatively, the raw materials may be charged in a caged-shape metal gauze and extracted, and thereafter the extraction residue can be collected. The purification after the solid-liquid separation can be carried out by cooling polymeric ingredients solubilized in the extract at a low temperature (preferably from 10°C or lower and 0°C or higher), aggregating and precipitating these ingredients by using persimmon tannin or a sedimentation agent by an ordinary method, and filtering the precipitates, to give a clear solution. The filtration may be preferably carried out by filtration with a 1 $\mu$m $\phi$- or 0.45 $\mu$m $\phi$ pore size membrane filter.

The protease used for the protease treatment, namely the protease used when the raw material marine alga is degraded by protease, is not particularly limited, and any of those originated from animals, plants and microorganisms can be used. The protease includes those originated from animals such as chymotrypsin, trypsin, pepsin and chymosin, those originated from plants such as papain and bromelain, and those originated from microorganisms such as bacteria, actynomycetes, *Aspergillus oryzae*, molds, and Basidiomycetes, including acidic, neutral and alkaline proteases. In addition, the proteases referred to herein encompass collagenase, esterase and keratinase. Also, at least one member selected from carboxypeptidase, aminopeptidase, lipase, pectinase, cellulase and hemicellulase may be used together with the protease.

When the marine alga is subjected to the protease treatment as a pretreatment, the amount of the protease used is not particularly limited. The amount of the protease is preferably from 0.01 to 10 parts by weight, more preferably from 0.05 to 5 parts by weight, per 100 parts by weight of the dry marine alga used. The solvent is not particularly limited as long as the desired enzymatic reaction can be carried out, and water or an aqueous solution of ethyl alcohol (concentration of ethyl alcohol: 0 to 50% by volume) can be preferably used. In addition, the reaction temperature is not particularly limited by the enzyme used. The reaction temperature is preferably from 30° to 120°C, and from the viewpoint of operability, the reaction temperature is more preferably from 50° to 100°C. The reaction time can be properly set depending upon the reaction temperature, and the reaction time is preferably from 0.1 to 32 hours, more preferably from 0.2 to 24 hours.

On the other hand, when the protease is added in the extraction step, and the extraction is carried out with the protease treatment, the amount of the protease used is not particularly limited. The amount of the protease is preferably from 0.01 to 10 parts by weight, more preferably from 0.05 to 5 parts by weight, per 100 parts by weight of the dry marine alga used.

The resulting marine alga extract is, for instance, sterilized with heating at 120°C for 20 seconds, thereafter concentrated, used directly as such or diluted with water, and further sterilized by filtration, and the resulting extract is

filled into a vessel. At this time, the filling may be carried out under nitrogen gas, and thereafter sterilized with heating at 90°C for 1 minute to give a manufactured article.

The shape of the extract according to the present invention is not particularly limited, and may be in the form of liquids, solids such as dry products, or powders.

Further, the fucoidan extracted under reduction can be separated from the marine alga extract of the present invention by a known method in a high yield. The method for separating the fucoidan from the marine alga extract is not particularly limited, as long as it is a known method for separating polymeric components. For instance, a marine alga is subjected to protease treatment in the presence of a reducing substance, the treated marine alga is then extracted with hot water or a solvent, and the extract is subjected to solid-liquid separation by means of a filter-press. The liquid portion is concentrated and desalted with an ultrafilter, and thereafter filtered with diatomaceous earth. Subsequently, the filtrate is concentrated with a concentrator, and sterilized in an autoclave at 121°C for 15 minutes. The resulting concentrate is lyophilized, whereby a fucoidan extracted under reduction of the present invention can be obtained.

Furthermore, the resulting fucoidan is degraded by a known method, whereby a degradation product of the fucoidan can be obtained. For instance, a degradation product of the fucoidan is prepared by the method according to WO 97/26896, WO 99/41288 or WO 00/50464, and the degradation product can be used in the same manner as the marine alga extract or the fucoidan of the present invention. The present invention also encompasses these degradation products of the fucoidan.

The process for manufacturing a food, beverage, seasoning, feed and medicament provided in the second embodiment of the present invention and its use embodiment are not particularly limited, each of which can be carried out in accordance with the above-mentioned food, beverage, feed and agent for maintaining homeostasis in the first embodiment of the present invention. On the other hand, the cosmetics which are also provided similarly in the second embodiment has known physiological actions of the fucoidan and/or the marine alga extract contained as an effective ingredient, for instance, an action for improving skin moisturizing ability or skin elasticity, an anti-aging action of skin, an anti-allergic action and the like. According to the cosmetics, there can be expected the effects of amelioration or prevention of wrinkles, improvement or sustenance of skin elasticity, amelioration or prevention of epidermal thickening and the like. Its manufacturing process and use embodiment are not particularly limited. For instance, there are the following embodiments.

In the cosmetics, the content of the fucoidan and/or marine alga extract of the present invention is usually preferably from 0.0001 to 20% by weight, more preferably from 0.001 to 5% by weight, especially preferably from 0.03 to 3% by weight.

In addition, as other components, various known ingredients in the field of cosmetics can be contained as desired. The other components include, for instance, moisturizing agents such as pyrrolidonecarboxylates; skin unlimbering agents such as liquid paraffins and vaseline; vitamins such as vitamin E; surfactants such as propylene glycol monostearate; emulsification stabilizers such as stearyl alcohol; anticorrosive agents; pigments; antioxidants; ultraviolet absorbents; and the like. These ingredients may be contained in an amount in which the effects of the components can be expected as desired within the range so as not to inhibit the exhibition of the desired effects of the present invention.

The form of the cosmetics is not particularly limited, and the preferable form includes, for instance, a lotion, a milky lotion, cream, a facial pack, a bathing agent, a facial cleansing agent, a bathing soap, a bath detergent and an ointment.

The cosmetics can be appropriately manufactured in accordance with a method known in the field of cosmetics using the fucoidan and/or marine alga extract according to the present invention and the above-mentioned other ingredients as desired as the raw materials. In addition, the cosmetics are used depending upon the shape of the cosmetics, so that the desired effects are appropriately obtained. For instance, if the cosmetics are a lotion, which is applied to, for instance, an entire facial surface of human, the lotion is used in an amount of preferably from 0.01 to 5 g or so, per use, whereby giving stretch and gloss to skin and obtaining lustrous skin, thereby giving a cosmeticizing effect.

Furthermore, the present invention provides. a preferred process for manufacturing a food or the like according to the present invention. In other words, there are provided processes for manufacturing a food, beverage, seasoning or feed; a process for manufacturing cosmetics; and a process for manufacturing a medicament (preferably a medicament for lowering cholesterol, a medicament for clarification of blood, a medicament for anti-coagulation, a medicament for anti-cancer, a medicament for anti-AIDS virus, or a medicament for anti-ulcer), characterized in that the processes comprises the step of extracting a marine alga in the presence of a reducing substance in the process for preparing a fucoidan of the present invention, and/or the step of extracting a marine alga in the presence of a reducing substance in the process for preparing a marine alga of the present invention. Each of the above processes comprises the step of preparing the fucoidan and/or marine alga extract of the present invention in any of the stages in known manufacturing processes for a food, cosmetics, a medicament and the like. The preparation of the fucoidan and/or

marine..alga extract which is a raw material for the above-mentioned food or the like, acting as an effective ingredient, and the preparation of the final manufacturing article food are continuously carried out, whereby the food or the like of the present invention can be more efficiently manufactured.

There are no cases of death when the fucoidan, a degradation product thereof and/or a salt thereof, and the marine alga extract, each especially having an action for maintaining homeostasis in a living body, used as the effective ingredient in the present invention is orally administered to a mouse once at 2 g/kg.

EXAMPLES

The present invention will be more concretely described by means of the following examples, without limiting the scope of the present invention thereto. Here, "%" in Examples means "% by weight" unless otherwise indicated.

Preparation Example 1

(1) *Kjellmaniella crassifolia* was sufficiently dried, and thereafter 20 kg of the dried product was powdered with a free mill (manufactured by Nara Kikai Seisakusho).

In 900 liters of tap water was dissolved 7.3 kg of calcium chloride dihydrate (manufactured by Nippon Soda Co., Ltd.), and 20 kg of the powdered product of *Kjellmaniella crassifolia* was then mixed therewith. The resulting mixture was heated for 40 minutes by blowing steam until the liquid temperature was raised from 12° to 90°C. Thereafter, the mixture was kept at 90° to 95°C for 1 hour under stirring, and then cooled, to give 1100 liters of a cooled product.

Subsequently, the cooled product was subjected to solid-liquid separation with a solid-liquid separator (manufactured by West Farrier Separator, Model: CNA), to give about 900 liters of supernatant after solid-liquid separation.

The amount 360 liters of the supernatant after solid-liquid separation was concentrated up to a volume of 20 liters with FE10-FC-FUS0382 (fraction molecular weight: 30000) manufactured by DAICEL CHEMICAL INDUSTRIES, LTD. Thereafter, the steps of adding 20 liters of tap water and again concentrating the resulting liquid mixture up to a volume of 20 liters were repeated 5 times, and the concentrate was subjected to a desalting treatment, to give 25 liters of an extract derived from *Kjellmaniella crassifolia*.

One liter of the extract was lyophilized, to give 13 g of a dried product of a fucoidan derived from *Kjellmaniella crassifolia*.

(2) Seven grams of the dried product of the fucoidan described in item (1) of Preparation Example 1 was dissolved in 700 ml of a 20 mM imidazole buffer (pH 8.0) containing 50 mM sodium chloride and 10% ethanol, and insoluble substances were removed by centrifugation. The supernatant after centrifugation was applied onto a DEAE-Cellulofine A-800 column (ϕ 11.4 cm x 48 cm) equilibrated with the same buffer, and then washed with the same buffer. The elution was carried out with a concentration gradient of from 50 mM to 1.95 M sodium chloride (250 ml per fraction). A total sugar level and an uronic acid content were determined by the phenol-sulfuric acid method and the carbazole-sulfuric acid method, to give Fractions 43 to 49, Fractions 50 to 55, and Fractions 56 to 67, in the order of elution. Next, these fractions were desalted by electrodialysis, and thereafter lyophilized, to give each of Fraction I (340 mg) from Fractions 43 to 49, Fraction II (870 mg) from Fractions 50 to 55, and Fraction III (2.64 g) from Fractions 56 to 67.

Figure 1 shows an elution pattern of the fucoidan derived from *Kjellmaniella crassifolia* on the DEAE-Cellulofine A-800 column. In Figure 1, the axis of ordinates is the absorbance at 530 nm as determined by the carbazole-sulfuric acid method (solid circles in the figure), the absorbance at 480 nm as determined by the phenol-sulfuric acid method (open circles in the figure), and the electric conductivity (mS/cm: open squares in the figure), and the axis of abscissas is the fraction number. In the figure, the front peak shows U-fucoidan, and the back peak shows F-fucoidan.

(3) A sulfated fucose-containing polysaccharide fraction was prepared from *Kjellmaniella crassifolia*. Specifically, 2 kg of commercially available dried *Kjellmaniella crassifolia* was powdered with a cutter mill (manufactured by Masuko Sangyo) fitted with a screen having a hole diameter of 1 mm, and the resulting powders were suspended in 20 liters of 80% ethanol. The suspension was then stirred at 25°C for 3 hours and filtered with a filter paper. The residue obtained was suspended in 40 liters of a 30 mM sodium phosphate buffer (pH 6.5) containing 100 mM sodium chloride, the suspension was allowed to stand at 95°C for 2 hours. Thereafter, the suspension was filtered with a stainless screen having a hole diameter of 106 µm. To the resulting filtrate were added 200 g of activated carbon, 4.5 liters of ethanol, and 12000 U of alginic acid lyase K (manufactured by Nagase Seikagaku Kogyo), and the mixture was stirred at 25°C for 20 hours and then centrifuged. The resulting supernatant was

concentrated to a volume of 4 liters with an ultrafilter equipped with holofiber having an excluding molecular weight of 100000, and thereafter insoluble substances were removed by centrifugation. The resulting solution was allowed to stand at 5°C for 24 hours, and precipitates formed were removed by centrifugation. The solvent for the resulting supernatant was exchanged for 100 mM sodium chloride with an ultrafilter. This solution was cooled to 4°C or lower, thereafter the pH of the solution was adjusted to 2.0 with hydrochloric acid, and precipitates formed were removed by centrifugation. The pH of the resulting supernatant was adjusted to 8.0 with sodium hydroxide, and the resulting solution was concentrated to a volume of 4 liters. Thereafter, the solvent for the resulting solution was exchanged for 20 mM sodium chloride with an ultrafilter. After insoluble substances in this solution were removed by centrifugation, the resulting supernatant was lyophilized, to give 76 g of a dried product of a fucoidan derived from *Kjellmaniella crassifolia.*

Preparation Example 2

(1) A 2-liter Erlenmeyer flask was charged with 600 ml of a culture medium comprising an artificial sea water (manufactured by Jamarin Laboratory), pH 8.2, containing 0.25% glucose, 1.0% peptone, and 0.05% yeast extract, and then sterilized (at 120°C for 20 minutes). *Alteromonas* sp. SN-1009 (FERM BP-5747) was inoculated into the culture medium, and cultured at 25°C for 26 hours, to give a seed culture medium. A 30-liter jar fermentor was charged with 20 liters of a culture medium comprising an artificial sea water (pH 8.0) containing 1.0% peptone, 0.02% yeast extract, 0.2% fucoidan described in item (2) of Preparation Example 2 described below, and 0.01% defoaming agent (manufactured by Shin-Etsu Chemical Co., Ltd., KM70), and sterilized at 120°C for 20 minutes. After cooling, the 30-liter jar fermentor was charged with 600 ml of the above-mentioned seed culture medium, and cultured at 24°C for 24 hours under the conditions of 10 liters of aeration per minute and a stirring rate of 250 rpm. After termination of the culture, the culture medium was centrifuged, to give cells and culture supernatant. This culture supernatant was concentrated with an ultrafilter equipped with holofiber having an excluding molecular weight of 10000, and the concentrate was then subjected to salting out with an 85% saturated ammonium sulfate. Precipitates formed were harvested by centrifugation, and sufficiently dialyzed against a 20 mM Tris-HCl buffer (pH 8.2) containing an artificial sea water at a one-tenth concentration, to give 600 ml of a solution of an endo-sulfated polysaccharide-degrading enzyme (F-fucoidan-specific degradation enzyme), selectively acting on the sulfated polysaccharide.

(2) Two kilograms of dried *Kjellmaniella crassifolia* was powdered with a cutter mill (manufactured by Masuko Sangyo) fitted with a screen having a diameter of 1 mm, and the resulting seaweed chips were suspended in 20 liters of 80% ethanol. The suspension was stirred at 25°C for 3 hours and filtered with a filter paper, and thereafter the residue was sufficiently washed. The residue obtained was suspended in 40 liters of a 20 mM sodium phosphate buffer, pH 6.5, containing 50 mM sodium chloride, which had been heated to 95°C, and the resulting suspension was kept at 95°C for 2 hours, with occasionally stirring, to extract a sulfated polysaccharide.

The suspension in the extract was filtered, to give a filtrate. Thereafter, the filtration residue was washed with 3.5 liters of 100 mM sodium chloride, to give an additional filtrate.

Both filtrates were combined, and then the temperature was lowered to 30°C. After 3000 U of alginic acid lyase (manufactured by Nagase Seikagaku Kogyo) was added to the resulting mixture, 4 liters of ethanol was added thereto. The resulting mixture was stirred at 25°C for 24 hours. Next, the mixture was centrifuged, and the resulting supernatant was concentrated up to a volume of 4 liters with an ultrafilter equipped with holofiber having an excluding molecular weight of 100000. Further, the ultrafiltration was continued with 100 mM sodium chloride containing 10% ethanol until a colored substance was no longer filtered.

Precipitates formed in a non-filtrate solution were removed by centrifugation, and the temperature of this supernatant was lowered to 5°C. The pH was adjusted to 2.0 with 0.5 N hydrochloric acid, and thereafter the formed precipitates such as a protein were removed by centrifugation. The pH of the resulting supernatant was rapidly adjusted to 8.0 with 1 N sodium hydroxide.

Next, an ultrafiltration was carried out with an ultrafilter equipped with holofiber having an excluding molecular weight of 100000, and the solvent was completely substituted with 20 mM sodium chloride (pH 8.0). Thereafter, the pH was again adjusted to 8.0, and the resulting mixture was centrifuged and then lyophilized, to give about 95 g of a sulfated polysaccharide.

(3) Two kilograms of dried *Kjellmaniella crassifolia* was powdered with a cutter mill fitted with a screen having a diameter of 1 mm, and the resulting seaweed chips were suspended in 20 liters of 80% ethanol. The suspension was stirred at 25°C for 3 hours and filtered with a filter paper, and thereafter the residue was sufficiently washed. The residue obtained was suspended in 20 liters of a 50 mM imidazole buffer (pH 8.2) containing 30 ml of F-fucoidan-specific degradation enzyme prepared in item (1) of Preparation Example 2 described above, 10% ethanol, 100 mM sodium chloride and 50 mM calcium chloride, and the resulting suspension was stirred at 25°C for 48 hours. This suspension was filtered with a stainless screen having a screen-opening diameter of 32 µm, and the residue was washed with 10% ethanol containing 50 mM calcium chloride. Further, the residue was suspended in 10 liters of 10% ethanol containing 50 mM calcium chloride, and the suspension was stirred for 3 hours, and thereafter filtered with the stainless screen, and the residue was washed. Further, the residue was suspended under the same conditions, and the suspension was then stirred for 16 hours. The suspension was filtered with the stainless screen having a diameter of 32 µm, and the residue was washed in the same manner.

The filtrate and the washings thus obtained were collected, and the combined mixture was subjected to ultrafiltration with an ultrafilter equipped with holofiber having an excluding molecular weight of 3000, thereby separating a filtered solution from a non-filtered solution. This filtered solution was concentrated to a volume of about 3 liters with a rotary evaporator, and thereafter the concentrate was centrifuged, to give supernatant. The resulting supernatant was desalted with an electric dialyzer equipped with a membrane having an excluding molecular weight of 300. To this solution was added calcium acetate so as to give a concentration of 0.1 M, and precipitates formed were removed by centrifugation. This supernatant was applied onto a DEAE-Cellulofine column (amount of resin: 4 liters) equilibrated with 50 mM calcium acetate, and sufficiently washed with 50 mM calcium acetate and 50 mM sodium chloride. Thereafter, the elution was carried out with a gradient of from 50 mM to 800 mM sodium chloride. The eluate at this time was collected 500 ml per fraction. The collected fraction was analyzed by cellulose acetate membrane electrophoresis [*Analytical Biochemistry*, **37**, 197-202 (1970)]. As a result, a sulfated saccharide which was eluted at a concentration of about 0.4 M sodium chloride (Proximity of Fraction No. 63) was homogeneous. Then, a solution of Fraction No. 63 was firstly concentrated to a volume of 150 ml, and thereafter sodium chloride was added so as to give a concentration of 4 M. The resulting solution was applied onto a Phenyl-Cellulofine column (amount of resin: 200 ml) previously equilibrated with 4 M sodium chloride, and sufficiently washed with 4 M sodium chloride. Non-adsorbent sulfated saccharide fractions were collected, and desalted with an electrodialyzer equipped with a membrane having an excluding molecular weight of 300, to give 505 ml of a desalted solution. Forty milliliters of the desalted solution obtained was applied onto a Cellulofine GCL-90 column (4.1 cm x 87 cm) equilibrated with 0.2 M sodium chloride containing 10% ethanol, to perform gel filtration. The collection was performed at 9.2 ml per fraction. All of the fractions were analyzed for a total sugar level by the phenol-sulfuric acid method [*Analytical Chemistry*, **28**, 350 (1956)].

As a result, since the sulfated saccharide formed a single peak, Fraction Nos. 63 to 70, which were fractions corresponding to a central part of the peak were collected. The combined fraction was desalted with an electrodialyzer equipped with a membrane having an excluding molecular weight of 300, and thereafter lyophilized, to give 112 mg of a dried product of the compound represented by the following formula (V). The compound is referred to as 7-12SFd-F.

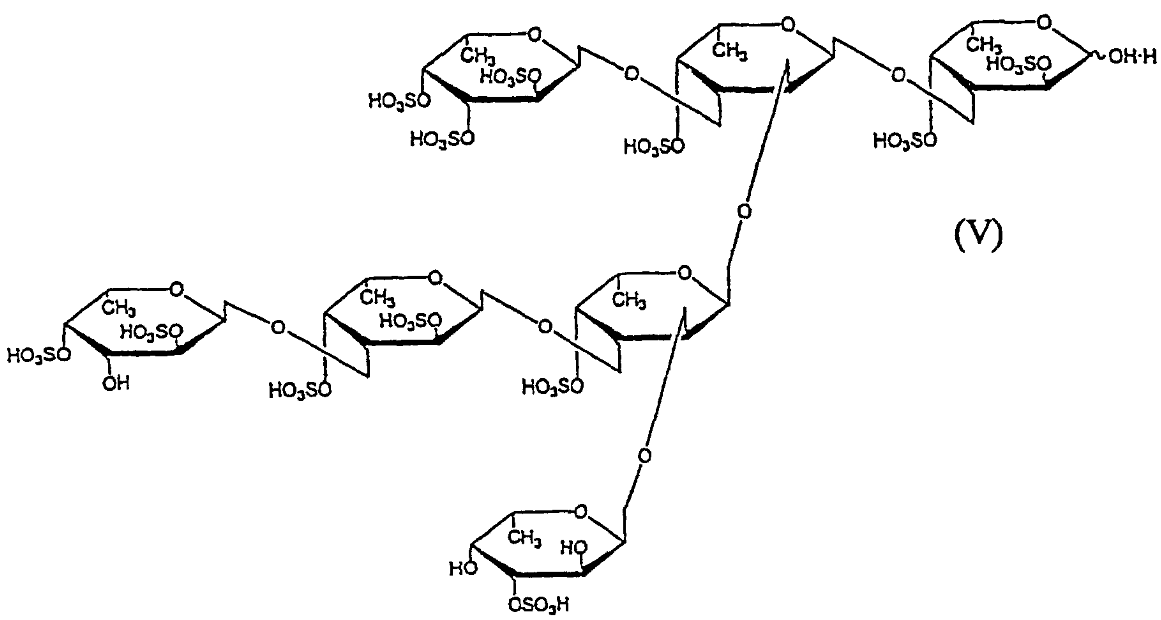

(V)

(4) Sufficiently dried *Kjellmaniella crassifolia* was powdered with a free mill (manufactured by Nara Kikai Seisakusho), and the powdered *Kjellmaniella crassifolia* was extracted with hot water at 95°C for 2 hours. Subsequently, the extract was subjected to solid-liquid separation with a decanter, and thereafter the liquid obtained was concentrated with an ultrafiltration membrane having an excluding molecular weight of 30000, to give a fucoidan extract.

(5) A culture of *E. coli* BL21 (DE3)/pEFDAII103 comprising the endo-sulfated polysaccharide degrading enzyme (F-fucoidan-specific degradation enzyme) disclosed in WO 99/11797 was concentrated with an ultrafiltration membrane having an excluding molecular weight of 10000, and the resulting concentrate was used as an F-fucoidan-specific degradation enzyme solution. The fucoidan extract described in item (4) of Preparation Example 2 and the F-fucoidan-specific degradation enzyme solution were added to 25 mM boric acid/sodium hydroxide buffer (pH 7.5) containing 50 mM calcium chloride and 300 mM sodium chloride, and the mixture was reacted at 37°C for 19 hours.

The reaction solution was concentrated with an ultrafiltration membrane having an excluding molecular weight of 10000, and the filtrate was then further concentrated with a reverse osmotic condenser (manufactured by Toray Industries, Inc.). The resulting concentrate was desalted with an electrodialyzer (manufactured by ASAHI KASEI CORPORATION).

The resulting desalted solution was subjected to chromatography by sequentially applying onto a DE 52 (manufactured by Whatmann), a DEAE-Sepharose column (manufactured by Amersham-Pharmacia Biotech) and a polyethyleneimine gel column (manufactured by Yamazen).

The resulting sulfated saccharide fraction was desalted with the electrodialyzer, and then subjected to sterilization by filtration and lyophilized, to give a dried product preparation of the compound 7-12SFd-F represented by the above-mentioned formula (V).

(6) Ninety-eight milligrams of F-fucoidan prepared according to the method described in item (2) of Preparation Example 1 was dissolved in 5 ml of DMSO, and 980 mg of piperidine sulfate was added thereto at room temperature. Thereafter, the resulting mixture was stirred at 80°C for 2 hours. After cooling the reaction solution, the reaction solution was dialyzed for 2 days with a dialyzing membrane having an excluding molecular weight of 1000. The retentate was applied onto a cation exchange column [Amberlite IRA-120 ($Na^+$)], and thereafter the eluate was dried in vacuo, to give 98 mg of highly-sulfated F-fucoidan.

(7) Thirty-four milligrams of 7-12SFd-F prepared according to the method described in item (3) of Preparation Example 2 was dissolved in 4 ml of DMSO, and thereafter the same procedures as those in item (6) of Preparation Example 2 were carried out, to give 34 mg of highly-sulfated 7-12SFd-F.

<u>Preparation Example 3</u>

(1) Two kilograms of dried *Kjellmaniella crassifolia* was powdered with a cutter mill (manufactured by Masuko Sangyo) fitted with a screen having a hole diameter of 1 mm. After the powders were stirred in 20 liters of 80% ethanol at 25°C for 3 hours, the mixture was filtered, and the residue was washed. The resulting residue was suspended in 20 liters of a 30 mM imidazole buffer (pH 8.2) containing 50 mM calcium chloride, 100 mM sodium chloride, 10% ethanol, and 1 U of *Alteromonas* sp. SN-1009-derived F-fucoidan-specific degradation enzyme prepared in item (1) of Preparation Example 2. The resulting suspension was stirred at 25°C for 2 days, and thereafter filtered with a stainless screen having a hole diameter of 32 μm, and the residue was washed. The resulting residue was suspended in 40 liters of a sodium phosphate buffer (pH 6.6) containing 100 mM sodium chloride, 10% ethanol and 4 g of an alginic acid lyase (manufactured by Nagase Seikagaku Kogyo). The resulting suspension was stirred at 25°C for 4 days, and thereafter-centrifuged, to give supernatant. In order to remove low-molecular weight products of alginic acid contained in the supernatant obtained, the supernatant was concentrated to a volume of 2 liters with an ultrafilter equipped with holofiber having an excluding molecular weight of 100000, and thereafter the solvent was exchanged for 100 mM sodium chloride containing 10% ethanol. To this solution was added an equivolume of 400 mM calcium acetate, the resulting mixture was stirred, and thereafter the mixture was centrifuged. The pH of the resulting supernatant was adjusted to 2.0 with 1 N hydrochloric acid, with cooling on ice. Precipitates formed were removed by centrifugation, and the pH of the resulting supernatant was adjusted to 8.0 with 1 N sodium hydroxide. This solution was concentrated to a volume of 1 liter by ultrafiltration, and thereafter the solvent of the concentrate was exchanged for 100 mM sodium chloride. Precipitates formed at this time were removed by centrifugation. In order to remove hydrophobic substances in the resulting supernatant, sodium chloride was added to the supernatant so as to give a concentration of 1 M, and the resulting mixture was applied onto a 3-liter Phenyl-Cellulofine column (manufactured by Seikagaku Corporation) equilibrated with 1 M sodium chloride, to collect an effluent fraction. This fraction was concentrated with an ultrafilter, and thereafter the solvent was exchanged

for 20 mM sodium chloride. The resulting solution was lyophilized, and the weight of the lyophilized product was 29.3 g.

(2) Fifteen grams of the above-mentioned lyophilized product was dissolved in 1.5 liters of 50 mM Tris-HCl buffer containing 400 mM sodium chloride and 9 U of the endo-sulfated polysaccharide-degrading enzyme (U-fucoidan-specific degradation enzyme) obtained from the culture prepared by culturing *Flavobacterium* sp. SA-0082 (FERM BP-5402) disclosed in WO97/26896. After the resulting solution was subjected to the reaction-at 25°C for 6 days, the reaction mixture was concentrated to a volume of about 300 ml with an evaporator. The concentrate was placed in a dialyzing tube having an excluding molecular weight of 3500 and thoroughly dialyzed. The solution remaining in the dialysis tube was applied onto a 4-liter DEAE-Cellulofine A-800 column equilibrated with 50 mM sodium chloride, and sufficiently washed with 50 mM sodium chloride. Thereafter, the elution was carried out on a concentration gradient of from 50 to 650 mM sodium chloride. Further, the elution was sufficiently carried out with 650 mM sodium chloride. Among the eluted fractions, the fractions eluted with 650 mM sodium chloride were collected as a sulfated fucogalactan fraction, and concentrated with an ultrafilter having an excluding molecular weight of 100000. Thereafter, the solvent of the concentrate was substituted with 10 mM sodium chloride, and the resulting solution was lyophilized, to give 0.85 g of a lyophilized product of sulfated fucogalactan. The sulfated fucogalactan obtained (G-fucoidan) was found to contain galactose and fucose as constituting saccharides in a molar ratio of about 2:1.

Preparation Example 4

The fucoidan derived from *Kjellmaniella crassifolia* obtained in item (3) of Preparation Example 1 was treated with the U-fucoidan-specific degradation enzyme described in item (2) of Preparation Example 3, to prepare a degradation product.

Specifically, 16 ml of a 2.5% aqueous fucoidan solution, 12 ml of 50 mM phosphate buffer (pH 7.5), 4 ml of 4M sodium chloride and 8 ml of an aqueous solution of 32 mU/ml of the above-mentioned U-fucoidan-specific degradation enzyme were mixed, and the resulting mixture was reacted at 25°C for 48 hours.

The reaction solution was subjected to molecular weight fractionation by a Cellulofine GCL-300 column (manufactured by Seikagaku Corporation), and fractions of molecular weight of 2000 or less were collected. This fraction was desalted with a microacylizer G3 (manufactured by ASAHI KASEI CORPORATION), and thereafter separated into 3 fractions by a DEAE-Sepharose FF column. The fractions were desalted, and thereafter lyophilized, to give purified products in amounts of 41 mg, 69 mg and 9.6 mg, respectively. It was confirmed that these purified products have respective molecular weights of 564, 724, and 1128 according to mass spectrometry, and that these purified products are represented by the respective formulas (VI), (VII) and (VIII), as shown below, according to NMR analysis. These compounds are hereinafter referred to as 3-1S, 3-3S and 6-2SFd-U, respectively.

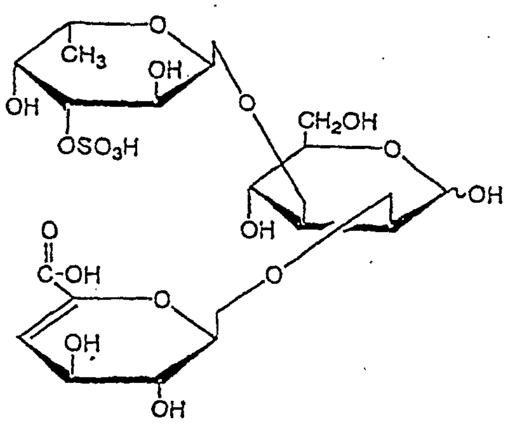

(VI)

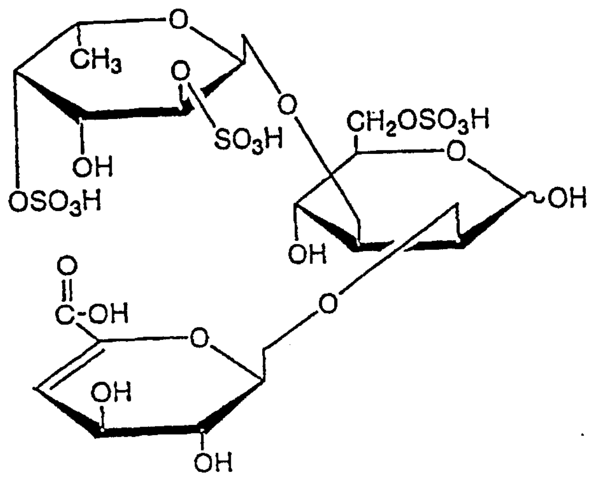

(VII)

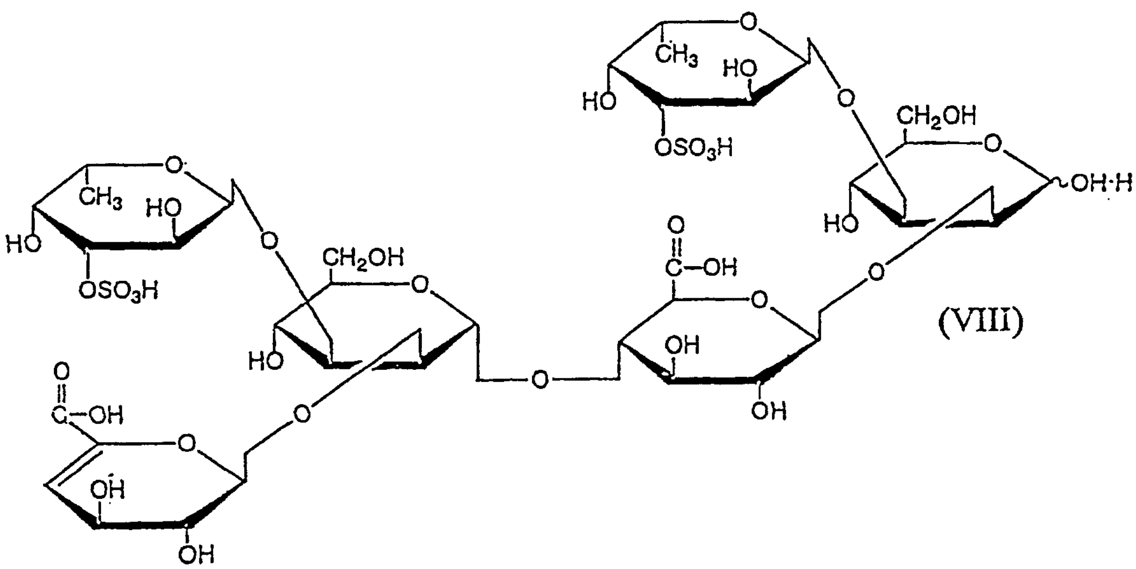

(VIII)

Preparation Example 5

One kilogram of a dried product of a commercially available sporophyll of *Undaria pinnatifida* was powdered with a cutter mill fitted with a screen having a hole diameter of 1 mm. Thereafter, the powdered sporophyll was suspended in 10 liters of 80% ethanol, and the suspension was stirred for 3 hours, and thereafter filtered with a filter paper, to give a residue. The residue was suspended in 20 liters of a 40 mM sodium phosphate buffer (pH 6.5) containing 50 mM sodium chloride, and treated at 95°C for 2 hours. The treated solution was cooled to 37°C, and thereafter ethanol was added thereto so as to give a concentration of 10%. 12000 U of a commercially available alginic acid lyase K (manufactured by Nagase Seikagaku Kogyo) was added thereto, and thereafter the mixture was stirred at room temperature for 24 hours. The resulting treated solution was centrifuged, and the supernatant was concentrated to a volume of 2 liters with an ultrafilter equipped with holofiber having an excluding molecular weight of 100000. Thereafter, precipitates formed were removed by centrifugation. The resulting supernatant was cooled to 5°C, and thereafter 0.5 N hydrochloric acid was added thereto to adjust the pH to 2.0. Subsequently, the resulting mixture was stirred for 30 minutes, and precipitates formed were removed by centrifugation. The pH of the supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained by centrifugation was lyophilized, to give 90.5 g of a fucoidan derived from sporophyll of *Undaria pinnatifida*.

Preparation Example 6

Two grams of the fucoidan derived from *Kjellmaniella crassifolia* prepared by the method described in item (1) of Preparation Example 1 was dissolved in 100 ml of water, and the pH of the solution was adjusted to 3 with citric acid. Thereafter, the resulting mixture was treated at 100°C for 3 hours, to give an acid-decomposed product of the fucoidan. This acid-decomposed product was subjected to molecular weight fractionation by gel filtration with a Cellulofine GCL-300 or a Cellulofine GCL-25, into fractions of a molecular weight exceeding 25000 (Fraction A), exceeding 10000 to 25000 (Fraction B), exceeding 5000 to 10000 (Fraction C), exceeding 2000 to 5000 (Fraction D), exceeding 500 to 2000 (Fraction E) and 500 or less (Fraction F). Further, each of these fractions and the acid-decomposed product were desalted, and then lyophilized, to give each fraction of the acid-decomposed product, and the acid-decomposed product.

Preparation Example 7

Five kilograms of a commercially available, salt-preserved *Nemacystus decipiens* was mixed with 20 liters of ethanol, and the salt-preserved *Nemacystus decipiens* in ethanol was cut into thin pieces with scissors. The resulting mixture was allowed to stand overnight, and then filtered with a filter paper. The resulting residue was suspended in 12.5 liters of water, and the suspension was kept at 95°C for 2 hours. After the suspension was filtered with a filter paper, 2600 ml of a 2.5% cetyl pyridinium chloride solution containing 350 mM sodium chloride was added thereto, and the resulting mixture was allowed to stand for 3 days. The supernatant portion was discarded, the precipitate portion was centrifuged, and the supernatant was also discarded. To the precipitates obtained was added 2.5 liters of 350 mM sodium chloride, and thereafter the mixture was homogenized with a homogenizer and centrifuged. The washing procedures were repeated 3 times. Four-hundred milliliters of 400 mM sodium chloride was added to the precipitates obtained. Thereafter, the mixture was homogenized with a homogenizer, and ethanol was added thereto so as to give a concentration of 80%. The mixture was stirred for 30 minutes, and then filtered with a filter paper. Five hundred milliliters of 80% ethanol saturated with sodium chloride was added to the residue obtained, and thereafter the mixture was homogenized with a homogenizer. Ethanol saturated with sodium chloride was added to make up a volume of 1 liter, and the mixture was stirred for 30 minutes and then filtered with a filter paper. The washing steps were repeated until the absorbance at 260 nm of the filtrate became substantially 0 (zero) (usually 5 times). The residue obtained was dissolved in 1.5 liters of 2 M sodium chloride, and thereafter insoluble substances were removed by centrifugation. The resulting solution was allowed to be effluent through a column containing 100 ml of DEAE-Cellulofine A-800 equilibrated with 2 M sodium chloride. Effluent fractions were concentrated to a volume of 2 liters with an ultrafilter equipped with holofiber having an excluding molecular weight of 100000, and thereafter the solvent was substituted with 2 mM sodium chloride by an ultrafilter. This solution was centrifuged, and the resulting supernatant was lyophilized, to give 22.9 g of a fucoidan derived from *Nemacystus decipiens*.

Preparation Example 8

Five kilograms of *stichopus* were dissected, and the organs were removed to collect somatic layers. Five-hundred milliliters of acetone was added per 200 g of the wet weight of the somatic layers, and the mixture was treated with a homogenizer. Thereafter, the homogenate was filtered, and the residue was washed with acetone until no more colored substances remained. This residue was dried with suction, to give 140 g of a dried product. To this dried product was added 2.8 liters of a 0.4 M aqueous sodium chloride, and the mixture was kept at 100°C for 1 hour. Thereafter, the mixture was filtered, and the residue was sufficiently washed with a 0.4 M aqueous sodium chloride, to give 3.7 liters of an extract. To this extract was added 5% cetyl pyridinium chloride until no more precipitates were formed, and the formed precipitates were harvested by centrifugation. The precipitates were suspended in a 0.4 M aqueous sodium chloride, and again centrifuged. One liter of a 4 M aqueous sodium chloride was added to the resulting precipitates, and the mixture was treated with a homogenizer. Thereafter, 4 liters of ethanol was added thereto with stirring, and the resulting mixture was stirred for 1 hour, and thereafter filtered, to give precipitates. The steps of suspending the

precipitates in 80% ethanol and thereafter filtering the suspension were repeated until the absorbance at 260 nm of the supernatant became substantially zero (0). The precipitates obtained were suspended in 2 liters of a 2 M aqueous sodium chloride, and insoluble substances were removed by centrifugation. The supernatant was ultrafiltered with an ultrafiltration having an excluding molecular weight of 30000, and completely desalted. Thereafter, the resulting product was lyophilized, to give 3.7 g of a fucoidan derived from sea cucumbers.

Preparation Example 9

Six-hundred and twenty-five grams of a commercially available, salt-preserved *Cladosiphon okamuranus* was suspended in 4375 ml of a 30 mM sodium phosphate buffer (pH 6.0), and the suspension was treated with a homogenizer at 8000 rotations per minute for 5 minutes. Thereafter, the homogenate was kept at 95°C for 1 hour, and centrifuged, to give supernatant. Ten grams of activated carbon was added to the resulting supernatant, and thereafter the resulting mixture was stirred for 30 minutes, and centrifuged, to give supernatant. The resulting supernatant was concentrated to a volume of 2 liters with an ultrafilter equipped with holofiber having an excluding molecular weight of 100000. Thereafter, the solvent was substituted with a 20 mM sodium chloride, and the resulting solution was lyophilized, to give 10.9 g of a dried product of a fucoidan fraction derived from *Cladosiphon okamuranus*.

Preparation Example 10

One kilogram of a dried product of powdered *Fucus vesiculosus* was suspended in 10 liters of 80% ethanol, and the suspension was stirred for 3 hours, and thereafter filtered with a filter paper, to give a residue. The residue was suspended in 30 liters of a 30 mM phosphate buffer (pH 6.0) containing 100 mM sodium chloride, and the resulting suspension was maintained at 95°C for 2 hours. After the suspension was cooled to 37°C, 100 g of activated carbon was added thereto, and the mixture was stirred for 30 minutes. After 3000 U of a commercially available alginic acid lyase K was added thereto, ethanol was added so as.to give a concentration of 10%, and the resulting mixture was stirred at room temperature for 24 hours. The resulting reaction solution was centrifuged, and the supernatant was concentrated to a volume of 2 liters with an ultrafilter equipped with holofiber having an excluding molecular weight of 100000. Thereafter, precipitates formed were removed by centrifugation, and this supernatant was ultrafiltered with adding a 30 mM phosphate buffer (pH 6.0) containing 100 mM sodium chloride, to remove a pigment. The non-filtered solution obtained was cooled to 5°C, and thereafter 0.5 N hydrochloric acid was added thereto to adjust the pH to 2.0. Subsequently, the resulting solution was stirred for 30 minutes, and precipitates formed were removed by centrifugation. The pH of the supernatant was adjusted to 8.0 with 0.5 N sodium hydroxide, and the solvent was substituted with 20 mM sodium chloride by ultrafiltration. The pH of the resulting solution was adjusted to 8.0, and thereafter the supernatant obtained after centrifugation was lyophilized, to give 71 g of a fucoidan derived from *Fucus vesiculosus.*

Example 1 Suppressive Effect of Hepatic Fibrosis by Fucoidan

Porcine serum (Gibco) was administered intraperitoneally to 7 week-old male SD rats twice a week at a dose of 0.5 ml/rat for 10 weeks to generate a model with hepatic fibrosis. A solution of the fucoidan derived from *Kjellmaniella crassifolia* described in item (1) of Preparation Example 1 was adjusted to a concentration of 0.5% with tap water, and given to the rats as drinking water 5 weeks from the initiation of the experiment. The control group was given tap water. The normal control group was similarly administered physiological saline in place of porcine serum.

The evaluation of the hepatic fibrosis was made using an increase in an amount of hydroxyproline, a major constituting amino acid of collagen, as an index. Specifically, the amount of hydroxyproline in hepatic tissues enucleated was determined, and expressed as a concentration per 1 g of the weight of the liver (μg/g liver) and as an amount of hydroxyproline to the whole liver (mg/whole liver). As a result, the group administered with the fucoidan derived from *Kjellmaniella crassifolia* showed a significantly reduced amount of hydroxyproline in the hepatic tissues, as compared to that of the control group. Further, the group administered with the fucoidan derived from *Kjellmaniella crassifolia* significantly suppressed an increase in the weight of the liver and an elevation in a ratio of the weight of the liver to the body weight due to accumulation of collagen, which was found in the control group. The results are shown in Tables 1 and 2. In addition, the enucleated hepatic tissues were observed. As a result, the incidence of fibrosis was not observed in the group administered with the fucoidan, as in the normal control group, while the incidence of fibrosis caused that the gloss on liver surface was lost and that ruggedness became distinct in the control group.

Table 1

| | n | Amount of Hydroxyproline* | |
|---|---|---|---|
| | | μg/g Liver | mg/Whole Liver |
| Control Group | 10 | 702 ± 94 | 11.7 ± 1.8 |
| Group Administered with Fucoidan | 10 | 336 ± 31** | 4.9 ± 0.5** |
| Normal Control Group | 4 | 164 ± 9 | 2.2 ± 0.0 |

* : Average Value ± Standard Error
**: $p < 0.01$ vs Control Group

Table 2

| | n | Body Weight (g) | Weight of Liver (g) | Ratio of Liver Weight to Body Weight (%) |
|---|---|---|---|---|
| Control Group | 10 | 463 ± 9 | 16.5 ± 0.7 | 3.5 ± 0.1 |
| Group Administered with Fucoidan | 10 | 461 ± 11 | 14.6 ± 0.4* | 3.2 ± 0.1** |
| Normal Control Group | 4 | 458 ± 14 | 13.2 ± 0.6 | 2.9 ± 0.1 |
| Average Value ± Standard Error | | | | |

* : $p < 0.05$ vs Control Group
** : $p < 0.01$ vs Control Group

Example 2 Suppressive Effect of Hepatic Fibrosis by 7-12SFd-F

Porcine serum (Gibco) was administered intraperitoneally to 7 week-old male SD rats twice a week at a dose of 0.5 ml/rat for 8 weeks to generate a model with hepatic fibrosis. The dried 7-12SFd-F preparation prepared in item (4) of Preparation Example 2 was diluted with distilled water, and given to the rats by forced oral administration at a dose of 100 or 30 mg/5 ml/kg every day after 4 weeks from the initiation of the experiment. The control group was given distilled water in the same manner. The normal control group was similarly administered with physiological saline in place of porcine serum.

The evaluation of the hepatic fibrosis was made by determining the amount of hydroxyproline in liver enucleated. The results are shown in Table 3.

As a result, the hepatic fibrosis (accumulation of collagen in the liver) was progressed by the administration of the porcine serum for 8 weeks, the amount of hydroxyproline in hepatic tissues and the concentration per unit weight of liver, increasing to about 4 times those of the normal liver. On the other hand, the amount of hydroxyproline in hepatic tissues and the concentration per unit weight of the liver were maintained at a level nearly to that on the fourth week of the porcine serum administration by orally administering 7-12SFd-F at a dose of 100 or 30 mg/kg every day from the fourth week of the porcine serum administration.

It was found from the above results that 7-12SFd-F suppressed the progression of the hepatic fibrosis.

In addition, the enucleated hepatic tissues were observed. As a result, the incidence of fibrosis was obviously suppressed in the group administered with the 7-12SFd-F, while incidence of fibrosis caused that the gloss on the liver surface was lost and ruggedness became distinct in the control group.

Table 3

| | n | Amount of Hydroxyproline* | |
|---|---|---|---|
| | | μg/g Liver | mg/Whole Liver |
| Control Group (8 weeks) | 11 | 286 ± 40 | 4.83 ± 0.71 |
| Control Group (4 weeks) | 8 | 193 ± 11 | 2.70 ± 0.17 |
| Group Administered with 7-12SFd-F 100 mg/kg | 8 | 199 ± 43 | 3.11 ± 0.79 |

* : Average Value ± Standard Error

Table 3 (continued)

| | | Amount of Hydroxyproline* | |
|---|---|---|---|
| | n | μg/g Liver | mg/Whole Liver |
| 30 mg/kg | 8 | 199 ± 34 | 3.08 ± 0.53 |
| Normal Control Group | 5 | 77 ± 5 | 1.14 ± 0.11 |

* : Average Value ± Standard Error

Example 3 Suppressive Effect of Hepatopathy Due to Alcohol-Uptake by Fucoidan

Five-week old male Crj:CD (SD) rats (Charles River Japan, INC.) were purchased, and the feed used was a liquid feed (manufactured by CLEA Japan). The fucoidan derived from *Kjellmaniella crassifolia* described in item (1) of Preparation Example 1 was dissolved in tap water, and the solution was orally administered to the rats (The doses are shown in Table 4). The control group was given tap water. Defining the day of initiation of giving the fucoidan derived from *Kjellmaniella crassifolia* as Day 0, ethanol was added to the CE-2 feed from the seventh day so as to have a final concentration of 5%, and the resulting feed was given to the rats as an alcoholic feed.

The evaluation of hepatopathy suppression was made by subjecting serum to serum biochemistry examination. Concretely, blood was taken on the 35th day, and heparinized. Thereafter, plasma was obtained by centrifugation, and the blood markers (GOT, GPT and γGTP) were determined by a serum biochemistry examination. In addition, the liver was enucleated, immobilized with a 10% neutral formalin buffer, embedded in paraffin and subjected to hematoxylin-eosin staining, and then pathologically observed. As the reagents for determinations of GOT, GPT and γGTP, there were used, respectively, S.TA-HRII GOT 7070, S.TA-HRII GPT 7070 and L Type γGTP, assay kits (all of them from Wako Pure Chemical Industries). The rats were fasted after blood was taken on the 35th day, and on the 36th day blood was taken from the abdominal aorta under ether anesthesia, and heparinized. After centrifugation, serum was obtained, and the blood markers (HDL, LDL and VLDL) were determined by a serum biochemistry examination. The determinations of HDL, LDL and VLDL were carried out by electrophoresis. Incidentally, during the test period, there were no differences between the groups in the amount of feed taken and the amount of drinking water taken.

As a result, by oral administration of the fucoidan derived from *Kjellmaniella crassifolia,* the elevation in GOT, GPT and γGTP by alcohol-uptake was suppressed, the increase in a ratio of VLDL, which is considered as so-called "baddy cholesterol" among the blood cholesterols, was suppressed, and the decrease in ratio of HDL which is considered as "goody cholesterol" was suppressed. Further, it was found from the pathological findings of the liver that damage, necrosis and diffuse proliferation of hepatocytes were obviously suppressed. The results are shown in Table 4.

It was found from the above results that the fucoidan derived from *Kjellmaniella crassifolia* suppressed hepatopathy due to alcohol-uptake.

Table 4

| Suppressive Effect of Hepatopathy Due to Alcohol-Uptake in Rat by Fucoidan Derived from *Kjellmaniella Crassifolia* | | | |
|---|---|---|---|
| Marker | Fucoidan Derived from *Kjellmaniella Crassifolia* (mg/rat/day) | | |
| | 0 | 0.5 | 5 |
| GOT (IU/L) | 108 ± 12 | 93 ± 5.5 | 84 ± 4.2 |
| GPT (IU/L) | 63 ± 8.5 | 51 ± 6.4 | 37 ± 6.7 |
| γGTP (IU/L) | 1.7 ± 0.39 | 1.4 ± 0.16 | 1.0 ± 0.35 |
| HDL (%) | 60 ± 1.8 | 62 ± 2.3 | 70 ± 7.4 |
| LDL (%) | 21 ± 2.0 | 23 ± 3.6 | 15 ± 6.5 |
| VLDL (%) | 16 ± 1.6 | 11 ± 0.75 | 13 ± 2.8 |
| Diffuse Hepatocyte Proliferation | 2/9 | 0/8 | 0/7 |
| Diffuse Hepatocyte Necrosis | 2/9 | 0/8 | 1/7 |
| Average Value ± Standard Error ($p < 0.05$) | | | |

Example 4 Suppressive Effect of Hepatopathy Due to Alcohol-Uptake by Fucoidan

Rats were reared in the same manner as in Example 3 until the 35th day, and then fasted. On the 36th day, blood was taken from the abdominal aorta under ether anesthesia, and heparinized. After centrifugation, serum was obtained, and triglyceride level was determined by a serum biochemistry examination. The triglyceride level was determined using a triglyceride E-Test Wako (manufactured by Wako Pure Chemical Industries). Incidentally, during the test period, there were no differences between the groups in the amount of feed taken and the amount of drinking water taken.

As a result, there is a strong tendency to lower the triglyceride level, as compared to that of the control group. The results are shown in Table 5.

It was found from the above results that the fucoidan derived from *Kjellmaniella crassifolia* suppressed hepatopathy due to alcohol-uptake.

Table 5

| Marker | Fucoidan Derived from *Kjellmaniella Crassifolia* (mg/rat/day) | | | |
|---|---|---|---|---|
| | 0 | 0.5 | 5 | 50 |
| Triglyceride Level | 25.5 ± 3.55 | 19.8 ± 3.1 | 22.5 ± 3.14 | 15.1 ± 1.86 |
| Average Value ± Standard Error ($p < 0.05$) | | | | |

Example 5 Suppressive Effect of Elevation in Blood Sugar Level by Fucoidan

Five-week old male Crj:CD (SD) rats (SPF animals) were purchased from Charles River Japan, INC. The fucoidan described in item (1) of Preparation Example 1 was dissolved in tap water, to prepare a fucoidan solution, and the rat was allowed to take the fucoidan solution *ad libitum.* At this time, the concentration of the fucoidan solution was set at 0.005%, 0.05% or 0.5%. Also, tap water was given to the control group. On the 10th day of rearing, a liquid feed (2 g of glucose/kg) alone was orally administered to animals which had been fasted for 18 to 20 hours since the previous day. Blood was taken from the tail vein before the administration of the feed, and 0.5, 1, 2 and 3 hours after the administration, and heparinized. Thereafter, plasma was obtained by centrifugation, and the blood sugar level was determined. The results are shown in Figure 2. Incidentally, the blood sugar level was calculated by defining the blood sugar level of the control rats before the glucose administration as 100%. Figure 2 is a graph showing a suppressive action for postcibal elevation in the blood sugar level in rats by the fucoidan, wherein the axis of ordinates is blood sugar level (%), and the axis of abscissas is the time passed (hours). From the figure, the suppressive action for elevating the blood sugar level by oral administration of the fucoidan was confirmed.

In addition, a comparison was made on a total blood sugar level up to 6 hours in the same manner. As a result, the group administered with the fucoidan obviously suppressed elevation in the blood sugar level was, as compared to that of the control.

Example 6 Suppressive Effect of Elevation in Neutral Fat Level in Blood by Fucoidan

An olive oil-added liquid feed was orally administered in the same manner as in Example 5 to the rats on the 19th day of rearing so as to have a concentration of olive oil at 5 ml/kg. Blood was taken before feeding, and 1 and 2 hours after feeding, and the triglyceride level was determined. The results are shown in Figure 3. Here, the triglyceride level was calculated by defining the triglyceride level of the control rats before giving the olive oil-added liquid feed as 100%. Figure 3 is a graph showing a suppressive action on postcibal elevation in the neutral fat level in blood in rats by the fucoidan, wherein the axis of ordinates is the triglyceride level (%) and the axis of abscissas is the time passed (hours). From the figure, the suppressive effect for elevating the neutral fat level in blood by oral administration of the fucoidan was found.

In addition, a comparison was made on a total triglyceride level in blood up to 6 hours in the same manner. As a result, the group administered with the fucoidan showed obvious suppression in the elevation in the total triglyceride level in blood, as compared to that of the control.

Example 7

Thirty grams of sporophyll of *Undaria pinnatifida* (dried product, powdered to a size of 5 mm ϕ-sieve-pass) was placed in 970 g of desalted water, and sodium ascorbate was added thereto as a reducing agent so as to have a concentration of 0.005% w/w, 0.01% w/w, 0.02% w/w, 0.05% w/w, 0.08% w/w, 0.1% w/w, 0.5% w/w or 1.0% w/w. As

to the control, sodium ascorbate was not added. The extraction was carried out at 95°C for 1 hour, with occasional gentle stirring. The solid-liquid separation was carried out by adding a filter aid (Celite) at a concentration of 2%, followed by filtration using a filter paper No. 2 (manufactured by Toyo Roshi K.K.) and then a membrane filter having a pore size of ϕ 0.45 µm, to give an extract of sporophyll of *Undaria pinnatifida*. The iodine content was determined and the sensory evaluation was carried out with the resulting extract.

The results are shown in Table 6. The sensory evaluation was carried out using 5-grade scores (from 1: excellent to 5: poor) by 10 panelists, and expressed as the average value.

Table 6: Iodine Content and Sensory Evaluation of Extract of Sporophyll of *Undaria Pinnatifida*

| Item | Reducing Agent Added (Sodium Ascorbate, % w/w) | | | | | | | | |
|---|---|---|---|---|---|---|---|---|---|
| | 0 Control | 0.005 | 0.01 | 0.02 | 0.05 | 0.08 | 0.1 | 0.5 | 1.0 |
| Iodine Content (mg%, w/v) | 5.8 | 5.5 | 5.4 | 5.4 | 4.9 | 4.6 | 4.5 | 4.4 | 5.0 |
| Transfer Ratio (%) | 100 | 95 | 93 | 91 | 84 | 79 | 78 | 76 | 86 |
| Sensory Evaluation | | | | | | | | | |
| Flavor | 3.0 | 2.4 | 2.3 | 2.1 | 1.9 | 2.0 | 2.0 | 2.2 | 2.3 |
| Taste | 3.2 | 2.7 | 2.4 | 1.8 | 1.7 | 1.9 | 1.9 | 2.0 | 3.2 |
| Color | 3.5 | 3.2 | 3.1 | 3.0 | 3.1 | 3.2 | 3.3 | 3.2 | 3.2 |
| Overall Evaluation | 3.3 | 2.8 | 2.5 | 2.3 | 2.2 | 2.4 | 2.4 | 2.5 | 2.5 |
| Remarks | *1 | *2 | *3 | *4 | *5 | *6 | *7 | *8 | *9 |

Note *1: Strong odor of a marine alga, strong bitter taste.
*2: Somewhat reduced odor of a marine alga, slight bitter taste.
*3: Rather weak odor of a marine alga, less bitter taste.
*4: Generally reduced odor, including odor of a marine alga, no bitter taste, refreshing taste, excellent aftertaste.
*5: The same as left (*4).
*6: The same as left (*4) with slightly reduced freshness.
*7: The same as left (*4) with slightly reduced freshness.
*8: The same as left (*4) with reduced freshness.
*9: Odor different from odor of a marine alga being generated, no bitter taste, monotonous taste, reduced freshness.

From Table 6, the extracts of sporophyll of *Undaria pinnatifida* extracted with adding a reducing agent showed reduced odor of a marine alga and remarkably reduced bitter taste, thereby improving aftertaste with refreshness, as compared to that of the control without addition of the reducing agent, especially in the cases where the amount of the reducing agent was added at a concentration of from 0.02% w/w to 1.0% w/w.

From the comparison on the sensory evaluation, in the cases where sodium ascorbate was added at a concentration of from 0.005% w/w to 1.0% w/w, the overall evaluation score of flavor, taste and color was from 2.8 to 2.2, in contrast to that of the control of 3.3. In addition, the concentration of a reducing agent sodium ascorbate which gave an overall evaluation score of 2.5 or less was from 0.01% w/w to 1.0% w/w. From these results, it was found that the preferred amount of the reducing agent to be added was from 0.005% w/w to 1.0% w/w, sensorially preferably from 0.01% w/w to 0.1% w/w. As to the color, any of the ascorbate-added group was lighter in color than the control group of green-brown or colorless. Also, as to the iodine content, the transfer to the extract was reduced by adding the reducing agent. The transfer of iodine could be reduced by 5% by the addition of the reducing agent at a concentration of 0.005%, and the transfer of iodine could be reduced by 7% or more by the addition of the reducing agent at a concentration of 0.01% or more when the iodine content of the case without the addition of the reducing agent is defined as 100% (see Transfer Ratio in the table). It is deduced that these results are correlated with reduction in bitter taste.

Example 8

Nine grams of sporophyll of *Undaria pinnatifida* (dried product, powdered to a size of 5 mm ϕ-sieve-pass) was placed in 201 g of desalted water, and sodium ascorbate was added thereto as a reducing agent so as to have a concentration of 0.02% w/w. By setting the extraction time at 1 hour, the extraction was carried out at various temperatures of 75°, 80°, 90°, 95°, 100°, 120° and 130°C. Each of the resulting filtrates was treated in the same manner as in Example 7, and the sensory evaluation was carried out. The results are shown in Table 7.

Table 7

| Study on Extraction Temperature | | | | | |
|---|---|---|---|---|---|
| Extraction | Sensory Evaluation | | | | Remarks |
| Temp. (°C) | Flavor | Taste | Color | Overall Evaluation | |
| 75 | 2.6 | 2.7 | 2.7 | 2.7 | Somewhat reduced odor of a marine alga, slightly unsatisfactory rich taste |
| 80 | 2.4 | 2.2 | 2.9 | 2.5 | Considerably reduced odor of a marine alga, reduced bitter taste, with rich taste |
| 90 | 2.3 | 1.8 | 3.0 | 2.4 | Generally reduced odor, including reduced odor of a marine alga, reduced bitter taste, refreshing, good aftertaste |
| 95 | 2.1 | 1.8 | 3.0 | 2.3 | Generally reduced odor, including reduced odor of a marine alga, reduced bitter taste, refreshing, good aftertaste |
| 100 | 2.1 | 1.8 | 3.0 | 2.3 | Generally reduced odor, including reduced odor of a marine alga, reduced bitter taste, refreshing, good aftertaste |
| 120 | 2.1 | 2.2 | 3.1 | 2.5 | Reduced odor, bitter taste slightly remaining |
| 130 | 2.4 | 2.4 | 3.3 | 2.7 | Odor different from odor of a marine alga being generated, bitter taste slightly remaining |

From Table 7, higher overall evaluation scores by the sensory evaluation were obtained when the extraction temperature for sporophyll of *Undaria pinnatifida* was from 75° to 100°C. Also, when the extraction temperature exceeded 100°C, the overall evaluation scores in the sensory evaluation were lower. When the extraction was carried out at 130°C, the overall evaluation scores were almost at the same level as those in which the extraction was carried out at 75°C. Therefore, it was found that the range of the extraction temperature was preferably from 75° to 130°C, and more preferably from 80° to 100°C from the viewpoint of quality on the sensory evaluation.

Example 9

Nine grams of sporophyll of *Undaria pinnatifida* (dried product, powdered to a size of 5 mm ϕ-sieve-pass) was placed in 201 g of desalted water, and sodium ascorbate was added thereto as a reducing agent so as to have a concentration of 0.02% w/w. By setting the extraction temperature at 95°C, the extraction was carried out for 0.5, 1, 3 or 5 hours. Each of the resulting filtrates was treated in the same manner as in Example 7, and the sensory evaluation was carried out. The results are shown in Table 8.

Table 8

| Study on Extraction Time | | | | | |
|---|---|---|---|---|---|
| Extraction | Sensory Evaluation | | | | Remarks |
| Time (hours) | Flavor | Taste | Color | Overall Evaluation | |
| 0.5 | 2.4 | 2.0 | 3.0 | 2.5 | Reduced odor of a marine alga, excellent deliciousness, bitter taste slightly remaining |
| 1 | 2.1 | 1.8 | 3.0 | 2.3 | Generally reduced odor, including odor of a marine alga, reduced bitter taste, refreshing, excellent aftertaste |
| 3 | 2.1 | 1.8 | 3.2 | 2.4 | Generally reduced odor, including odor of a marine alga, reduced bitter taste, refreshing, excellent aftertaste |
| 5 | 2.5 | 2.5 | 3.2 | 2.7 | Odor different from odor of a marine alga slightly generated, slight taste of roughness remaining in the mouth |

From Table 8, the extraction time of the sporophyll of *Undaria pinnatifida* for exhibiting the effects of reduced odor of a marine alga, excellent deliciousness and slightly remaining bitter taste was 0.5 hours. Also, the overall evaluation score by the sensory evaluation was the most excellent in the case of extraction for 1 hour, and the value became higher with a longer extraction time. The effect of reducing odor of a marine alga was confirmed even in the case of extraction for 5 hours. Therefore, it was found that the preferable extraction time was from 0.5 to 5 hours.

Example 10

The influence of pH was examined. According to Example 9, the extraction was carried out at 95°C for 1 hour with adjusting the pH of the extraction solvent to 3.0, 4.0, 5.0, 6.0 and 7.0 with citric acid or sodium bicarbonate. As a result, it was confirmed that all the extracts obtained showed reduction in odor of a marine alga. A comparison was not made on the taste because the taste was affected by citric acid or sodium bicarbonate added. Therefore, it was found that the preferred pH during the extraction was pH 3.0 to 7.0 from the viewpoint of reducing odor of a marine alga.

Example 11

The amount 0.6 kg of sporophyll of *Undaria pinnatifida* (dried product, powdered to a size of 5 mm ϕ-sieve-pass) was placed in 20 liters of desalted water, and a reducing agent sodium ascorbate was added thereto so as to have a concentration of 0.02% w/w to the desalted water. For the control, the reducing agent was not added. The resulting mixture was heated with stirring, kept at 95°C for 1 hour, and then cooled to room temperature. The filtration was carried out using a filter paper (ADVANTEC #327), a filter aid (Silika #600S, 20 g) as a precoat and a filter aid (Celite #545, 45 g) as a body-feed according to a conventional method, and then using a membrane filter having a pore size of ϕ 1 μm, to give 19.4 liters of a filtrate. The filtrate was sterilized at 120°C for 60 seconds, and the resulting sterilized solution was concentrated under reduced pressure (40°C, 750 mmHg), to give 2.6 liters (7.58-folds) of a concentrate (22.7%). A portion (1 liter) of the concentrate was lyophilized, to give 9 g of a powder. These procedures were carried out 5 times. The analyzed values for the resulting concentrate are shown in Table 9.

Table 9

| Analysis of Concentrate | | |
|---|---|---|
| Item | Inventive Product | Control |
| PH | 5.35 | 5.36 |
| Acidity (0.1 N NaOH ml/10 ml) | 0.24 | 0.23 |
| Formol Nitrogen (mg%,w/v) | 109.4 | 110.0 |
| Total Nitrogen (mg%, w/v) | 222.5 | 231.0 |
| Direct Sugar (mg%, w/v) | 0.24 | 0.24 |
| Total Reducing Sugar (mg%, w/v) | 1.78 | 1.80 |
| Fucoidan (%, w/v) | 2.88 | 2 |
| NaCl (%, w/v) | 3.48 | 3.41 |
| Iodine (mg%, w/v) | 4.15 | 4.95 |
| Sensory Evaluation with 7.7-Fold Dilution (volume ratio of distilled water to concentrate: 6.7:1) | Reduced odor of a marine alga, no sensible bitter taste, with fresh taste | Strong odor of a marine alga, strong bitter taste |

From Table 9, the concentrate of the extract from sporophyll of *Undaria pinnatifida* of the present invention had most of analyzed values of almost the same level as those of the control. However, as to the iodine, the content was 84% of that of the control, showing that a reduction effect by 15% or more was obtained. In the sensory evaluation for the solution diluted 7.7-folds with distilled water, the inventive product showed a marked effect in flavor and taste of reduced odor of a marine alga, fresh taste and no sensible bitter taste, as compared to the control.

Example 12

Nine grams of each of *Kjellmaniella crassifolia, Laminaria japonica,* and thallus of *Undaria pinnatifida* (each being a dried product, and powdered to a size of 5 mm ϕ-sieve-pass) was placed in 201 g of desalted water, and sodium ascorbate was added thereto as a reducing agent so as to have a concentration of 0.02% w/w. By setting the extraction temperature at 95°C, the extraction was carried out for 1 hour. The control was prepared in the same manner without addition of sodium ascorbate. Each of the resulting filtrates was treated in the same manner as in Example 7, and the sensory evaluation was carried out. The results are shown in Table 10.

Table 10

| Sensory Evaluation | | | | | | |
|---|---|---|---|---|---|---|
| | | Flavor | Taste | Color | Overall Evaluation | Remarks |
| *Kjellmaniella Crassifolia* | Inventive Product | 2.8 | 2.7 | 3.1 | 2.9 | Reduced odor of a marine alga, no bitter taste |
| | Control | 3.3 | 3.8 | 3.6 | 3.6 | Odor of a marine alga and bitter taste |
| *Laminaria Japonica* | Inventive Product | 2.7 | 2.5 | 2.7 | 2.6 | Reduced odor of a marine alga, excellent deliciousness |
| | Control | 3.4 | 3.2 | 3.3 | 3.3 | Odor of a marine alga and bitter taste slightly remaining |
| Thallus of *Undaria Pinnatifida* | Inventive Product | 2.4 | 2.4 | 2.9 | 2.6 | Reduced odor of a marine alga, no bitter taste |
| | Control | 3.0 | 3.2 | 3.5 | 3.2 | Odor of a marine alga, bitter taste |

From Table 10, in the extraction of *Kjellmaniella crassifolia, Laminaria japonica* and the thallus of *Undaria pinnatifida* in the presence of the reducing substance, the odor of a marine alga can be reduced and the bitter taste can be removed in the same manner as in the sporophyll of *Undaria pinnatifida*, thereby improving both the flavor and taste, and had fresh taste. The inventive products were extracts having favorable taste as food regardless of the kind of seaweed.

Example 13

To 4 g of *Kjellmaniella crassifolia* belonging to Phaeophyceae (dried product, powdered to a size of 5 mm-ϕ-sieve-pass) was added desalted water containing 100 mmol/liter of calcium chloride, and sodium ascorbate was added thereto as a reducing agent so as to have a concentration of 0.01% w/w, 0.05% w/w, 0.10% w/w, 0.20% w/w or 0.50% w/w. The extraction was carried out at 95°C for 2 hours, and thereafter the resulting extract was filtered with a filter paper No. 2 (Toyo Roshi K.K.). The fucoidan content of the filtrate was determined using a method of measuring L-fucose by the cysteine-sulfuric acid method. The control used was prepared without the addition of sodium ascorbate. The results are shown in Table 11.

Table 11

| Fucoidan Content | |
|---|---|
| Sodium Ascorbate (% w/w) | Concentration of Fucoidan (mg/ml filtrate) |
| Without Addition (Control) | 0.89 (100%) |
| 0.01 | 1.11 (125%) |
| 0.05 | 1.33 (149%) |
| 0.10 | 1.33 (149%) |
| 0.20 | 1.44 (161%) |
| 0.50 | 1.45 (162%) |

From Table 11, the extraction ratio of the fucoidan was increased 1.6 times that of the control at a concentration of from 0.01 to 0.50% w/w of sodium ascorbate used, so that the extraction efficiency of the fucoidan was improved in the presence of a reducing agent sodium ascorbate.

Next, a combination of use of sodium ascorbate with a protease treatment was studied. Specifically, 4 g of *Kjellmaniella crassifolia* belonging to Phaeophyceae (dried product, powdered to a size of 5 mm ϕ-sieve-pass) and 40 mg of papain derived from a plant (Nagase Seikagaku Kogyo) or SP-15FG derived from *Bacillus subtilis* (Nagase Seikagaku Kogyo), as a protease, were mixed with 100 ml of desalted water containing 100 mmol/liter of calcium chloride. The resulting mixture was subjected to an enzyme treatment, with gradually heating the mixture from 12° to 95°C for over a period of 2 hours. Thereafter, a reducing agent sodium ascorbate was added so as to have a concentration of 0.10% w/w in each case. As the control, one in which only the enzyme was not added was used. The extraction was carried out at 95°C for 2 hours. After the extraction, the filtration was carried out using a filter paper No. 2 (Toyo Roshi K.K.). Four milliliters of this filtrate was desalted and concentrated to a volume of 0.1 ml with an ultrafiltration membrane (excluding molecular weight of 10,000). Four milliliters of desalted water was added to the resulting desalted concentrate in each case. The resulting solution was again concentrated to a volume of 0.1 ml, and desalted water was added to the concentrate to make up to a volume of 1 ml. The fucoidan content and the Kjeldahl nitrogen content of this solution were determined. In addition, from the determination results, the fucoidan yield (%) per weight of the raw material *Kjellmaniella crassifolia* was calculated:

Fucoidan Yield (%) = (Weight (g) of Fucoidan in 1 ml of Concentrate × 25)/

(Weight (g) of Raw Material *Kjellmaniella crassifolia*) × 100.

The results are shown in Table 12.

Table 12

| Fucoidan Content, Nitrogen Content and Fucoidan Yield | | | | |
|---|---|---|---|---|
| Enzyme | Fucoidan Content (g/L) | Total Nitrogen (mg/L) | Specific Turbidity *660 nm/cm cell | Fucoidan Yield (%) |
| Control | 6.05 | 64.3 | 0.158 | 3.78 |
| Protease SP-15FG | 8.42 | 47.8 | 0.089 | 5.26 |
| Papain | 8.29 | 29.9 | 0.108 | 5.18 |

* : Determined using a filtrate obtained by filtering the solution through a filter paper No. 2 (Toyo Roshi K.K.)

It was confirmed from Table 12 that the extraction efficiency of the fucoidan was further improved by combining the enzyme protease treatment and the extraction with sodium ascorbate. In addition, proteins derived from *Kjellmaniella crassifolia* were made to have lower molecular weights (molecular weight of 10,000 or less), so that the proteins could be reduced by removing the proteins by ultrafiltration. The amount of the proteins was one-half that of the control or less in the case of the treatment with papain. Further, the turbidity of the control extract became clear by the enzyme protease treatment. From these results, it was possible to improve the yield and to reduce the proteins by extracting the fucoidan with the enzyme protease treatment and the extraction with sodium ascorbate, so that high purity fucoidan could be obtained.

Example 14

(1) Forty kilograms of *Kjellmaniella crassifolia* belonging to Phaeophyceae (dried product, powdered to a size of 5 mm-ϕ-sieve-pass), 15 kg of calcium chloride, 400 g of an enzyme papain (Nagase Seikagaku Kogyo), and 1 kg of sodium ascorbate were mixed with desalted water, to make up a volume of 1000 liters. The enzyme treatment and the extraction were carried out by raising the temperature from 15° to 60°C over a period of 80 minutes, and carrying out the reaction and extraction at 60°C for 60 minutes. Further, the extraction was carried out by raising the temperature from 60° to 95°C over a period of 80 minutes, and carrying out the extraction at 95°C for 120 minutes. Thereafter, the resulting extract was cooled to 9°C over a period of 24 hours, to give an inventive extracted product. The fucoidan content of this extracted product was determined, and the fucoidan yield (%) per raw material *Kjellmaniella crassifolia* was calculated:

Fucoidan Yield (%) = (Weight (kg) of Fucoidan in Extracted Product)/

(Weight (kg) of Raw Material *Kjellmaniella Crassifolia*) × 100.

Also, the control was obtained by a similar treatment without the addition of a reducing agent sodium ascorbate and an enzyme papain. The determination results are shown in Table 13.

Table 13

| Fucoidan Content of Extracted Product | | |
|---|---|---|
| | Inventive Extracted Product | Control Extracted Product |
| Weight of Fucoidan (kg) | 2.07 | 1.81 |
| Fucoidan Yield (%) | 5.18 | 4.53 |

(2) Subsequently, the inventive extracted product obtained in item (1) of Example 14 was subjected to solid-liquid separation using a filter press, to give 800 liters of a filtrate. Next, the resulting filtrate was subjected to an ultrafiltration treatment and a desalting treatment, to concentrate the filtrate to a volume of 80 liters. This concentrate was filtered with a filter using a filter paper and diatomaceous earth, to give 77 liters of a filtrate. Further, the filtrate was concentrated using a concentrator (EVAPOR, CEP-30S, heating temperature: 90°C, product temperature: 40° to 45°C), to give 6.8 liters of a concentrate, and the concentrate was autoclaved at 121°C for 15 minutes. The

sterilized solution was lyophilized, to give 780 g of a fucoidan. The control was obtained by a similar treatment without adding a reducing agent sodium ascorbate and an enzyme papain. The analyzed values for the lyophilized products of the inventive product and the control, and for the 1% w/v aqueous solutions (fucoidan solutions) thereof were shown in Table 14 and Table 15, respectively.

Table 14

| Analyzed Values for Lyophilized Product | | | |
|---|---|---|---|
| | | Inventive Product | Control |
| Water | (g/100 g) | 0.4 | 0.9 |
| Protein [a] | (g/100 g) | 2.6 | 9.0 |
| Lipid | (g/100 g) | 0.1 | 0.2 |
| Ash Content | (g/100 g) | 31.8 | 29.4 |

a: Kjeldahl nitrogen × 6.25 (protein factor)

Table 15

| Fucoidan Solution | | |
|---|---|---|
| | Inventive Product | Control |
| Fucoidan (mM) | 21.4 | 20.0 |
| Total Sugar (mM, calculated as fucose) | 27.8 | 23.5 |
| Sulfate Group (mM) | 34.2 | 28.5 |

It was confirmed from Table 14 that the extraction efficiency of the fucoidan was improved by a combination of the enzyme protease treatment and the extraction with adding sodium ascorbate. Further, as is clear from Table 14, the protein content of the inventive product was remarkably reduced. In addition, as is clear from Table 15, a highly purified fucoidan could be obtained according to the method of the present invention.

In addition, the color of the external appearance of the inventive product was examined. As a result, the inventive product was whitish green-brown, lighter in color than green-brown of the control. Specifically, a solution prepared by dissolving the inventive product or the control product in distilled water so as to have a fucoidan content of 2 g/L, and the absorbance for the solution was determined at 660 nm. As a result, it was found that the absorbances for the solution of the inventive product and the control were 0.017 and 0.036, respectively, so that the inventive product showed reduced green-colored components.

In addition, the degree of odor of a marine alga of the inventive product was also examined. As a result, the odor of a marine alga was reduced in the inventive product, as compared to that of the control. Specifically, a 0.5% w/v solution was prepared by dissolving the obtained control in distilled water at 20°C. The concentration of a solution of the inventive product showing odor of the same strength as that in the control was judged by 5 panelists. As a result, the concentration for the inventive product was 2.6%, so that it was found that the odor of a marine alga was reduced in the inventive product, as compared to that of the control.

Example 15

Each of the beverages containing the extract of sporophyll of *Undaria pinnatifida* having a formulation as shown in Table 16 was prepared using the concentrate of the extract of sporophyll of *Undaria pinnatifida* of the present invention or that of the control of Example 11.

Table 16

| Beverage Containing Extract of Sporophyll of *Undaria pinnatifida* | | |
|---|---|---|
| | Inventive Product (%) | Control (%) |
| Extract (Concentrate) of Sporophyll of *Undaria pinnatifida* Treated With Reducing Agent | 18 | 0 |
| Extract (Concentrate) of Sporophyll of *Undaria pinnatifida* Without Treatment with Reducing Agent | 0 | 18 |

Table 16 (continued)

| Beverage Containing Extract of Sporophyll of *Undaria pinnatifida* | | |
|---|---|---|
| | Inventive Product (%) | Control (%) |
| Trehalose | 2.5 | 2.5 |
| 1/5 Apple Juice | 1.7 | 1.7 |
| 1/5 Lemon Juice | 0.13 | 0.13 |
| Vitamin C | 0.04 | 0.04 |
| Water | Balance | Balance |
| pH | 4.0 | 4.0 |
| Acidity 0.1 N NaOH/20 ml | 3.72 | 3.72 |
| Brix | 5.5 | 5.5 |
| pH was adjusted with citric acid. | | |

Each of the beverages prepared according to the formulation in Table 16 was filled in a 200-ml can, and thermally sterilized at 115°C for 15 minutes, to prepare canned articles. The sensory evaluation was carried out in the same manner as in Example 7. Ten panelists judged with the 5-grade scores (from 1: excellent to 5: poor), and the average value was obtained. The results are shown in Table 17.

Table 17

| Sensory Evaluation | | |
|---|---|---|
| | Inventive Product | Control |
| Taste | 2.6 | 3.3 |
| Flavor | 2.4 | 3.4 |
| Color Tone | 2.6 | 2.9 |
| Overall Evaluation | 2.5 | 3.2 |

From Table 17, the beverage prepared with the concentrate of the extract of sporophyll of *Undaria pinnatifida* of the inventive product was odorless of a marine alga and no bitter taste, showing well-balanced sweetness, sourness and deliciousness, thereby providing a seaweed beverage with novel flavor and refreshing aftertaste, as compared to that of the control. The control had strong odor of a marine alga from sporophyll of *Undaria pinnatifida*, bitter taste in the mouth, ill-balanced flavor and taste, and worsened aftertaste. Also, the inventive product had more excellent color tone, as compared to that of the control.

Example 16

A soup was prepared with an extract of sporophyll of *Undaria pinnatifida*. As the extract (concentrate) of sporophyll of *Undaria pinnatifida*, one in which the 3% extract of sporophyll of *Undaria pinnatifida* of Example 11 was concentrated 7.58-folds (corresponding to 22.7% extract of sporophyll of *Undaria pinnatifida*) was used. The extract subjected to the reduction treatment was used for the inventive product, and the extract without the treatment was used for the control. Table 18 shows the formulations of the soups containing the extract (concentrate) of sporophyll of *Undaria pinnatifida.* According to the formulations, each of the soups was prepared, and filled in a 200-ml can and thermally sterilized at 120°C for 15 minutes, to prepare canned articles. The sensory evaluation was carried out in the same manner as in Example 7. Ten panelists judged with the 5-grade scores (from 1: excellent to 5: poor), and the average value was obtained. The results are shown in Table 19.

Table 18

| Formulation of Soup Prepared with Extract (Concentrate) of Sporophyll of *Undaria pinnatifida* | | |
|---|---|---|
| | Inventive Product (%) | Control (%) |
| Extract (Concentrate) of Sporophyll of *Undaria pinnatifida* Treated with Reducing Agent | 10 | 0 |

Table 18 (continued)

| Formulation of Soup Prepared with Extract (Concentrate) of Sporophyll of *Undaria pinnatifida* | | |
|---|---|---|
| | Inventive Product (%) | Control (%) |
| Extract (Concentrate) of Sporophyll of *Undaria pinnatifida* Without Treatment with Reducing Agent | 0 | 10 |
| Low-Strength Agar * | 0.25 | 0.25 |
| Whey Minerals | 0.60 | 0.60 |
| Pork Extract | 0.13 | 0.13 |
| Sugar | 0.10 | 0.10 |
| Pepper | 0.0005 | 0.0005 |
| Vitamin C | 0.02 | 0.02 |
| Water | Balance | Balance |
| pH | 5.0 | 5.0 |

* Manufactured by Ina Food Industry Co., Ltd. pH was adjusted with citric acid or sodium citrate.

Table 19

| Sensory Evaluation | | | | |
|---|---|---|---|---|
| Soup | Item | | | Overall Evaluation |
| | Taste | Flavor | Color Tone | |
| Inventive Product | 2.9 | 2.7 | 3.0 | 2.9 |
| Control | 3.6 | 3.7 | 3.3 | 3.5 |

From Table 19, the inventive product had better aftertaste with refreshing taste than that of the control. Also, the inventive product had higher compatibility between the flavor and taste of the extract of sporophyll of *Undaria pinnatifida* and those of pork extract, so that a novel taste, which could not have been provided with the pork extract alone, could be provided. Therefore, the inventive product had well-balanced overall flavor and taste. The control had some odor of a marine alga remaining, a taste incompatible with the pork extract, with bitter taste slightly remaining. Therefore, the control had a tendency to lose its balance in the overall flavor and taste. As described above, from the aspect of cooking, it was revealed that the extract of sporophyll of *Undaria pinnatifida* of the inventive product was excellent as a seasoning judging from its characteristics of flavor and taste. In addition, the inventive product had better color tone than that of the control.

Example 17

A Japanese powdered sprinkle over rice (*furikake*) was prepared by mixing 2.4 kg of fish flour, 0.5 kg of table salt and 0.3 kg of sodium glutamate (total weight: 3.2 kg), adding thereto the lyophilized product of the extract of sporophyll of *Undaria pinnatifida* obtained in Example 11 in an amount of 5 g per 1 kg of the above Japanese powdered sprinkle for each of the inventive product or the control, or not adding the lyophilized product, and granulating the resulting mixture according to a conventional method. The resulting mixture was granulated according to the conventional method. The amount 1.2 kg of sesame was added to about 3.2 kg of each of the resulting granulated products, and thoroughly mixed, to prepare the Japanese powdered sprinkle. Each Japanese powdered sprinkle was sprinkled over cooked rice, and the sensory evaluation was carried out in the same manner as in Example 7. As a result, it was found that flavor and taste (*umami*) from the fish flour and sesame harmonize in the inventive product, as compared to that of the control, so that the inventive product serves as a "secret flavor" (*kakushiaji*). The control had slightly remaining odor of a marine alga, ill-balanced flavor between the fish flour and sesame, and bitter taste slightly remaining on the mouth. It was found that the inventive product makes the Japanese powdered sprinkle high quality.

Example 18

A Japanese powdered sprinkle over rice was prepared by mixing 2.4 kg of fish flour, 0.5 kg of table salt and 0.3 kg of monosodium glutamate (total: 3.2 kg), and adding the extract of sporophyll of *Undaria pinnatifida* obtained

in Example 11 for the inventive product and the control in an amount of 5 g per 1 kg of the above Japanese powdered sprinkle. In addition, 1 g of the fucoidan derived from *Kjellmaniella crassifolia* obtained in Example 14 was added only for the inventive product. Each Japanese powdered sprinkle was sprinkled over cooked rice, and the sensory evaluation was carried out in the same manner as in Example 7. As a result, flavor and taste (*umami*) from the fish flour and sesame harmonize in the inventive product, as compared to the control. Also, the inventive product had excellently well-balanced flavor, as compared to the control, thereby setting off the *Undaria pinnatifida* flavor. By combining the Japanese powdered sprinkle and the fucoidan, the quality of the Japanese powdered sprinkle was further improved.

Example 19

A confection was prepared by using the formulations as shown in Table 20 at a pressure of 3000 kg/cm$^2$ during tabletting according to the conventional method, using a tablet machine. In the inventive product, the extracted fucoidan obtained by the protease and reduction treatment was used, and in the control, the control fucoidan obtained in Example 14 was used. As a result, the inventive product had no odor of a marine alga when put in the mouth, an improved balance of the overall taste, and the inventive product was smooth on the tongue.

Table 20

| Table for Formulation | | | |
|---|---|---|---|
| | | Inventive Product | Control Product |
| Control Fucoidan | (mg) | 0 | 100 |
| Inventive Fucoidan | (mg) | 100 | 0 |
| Dextrin | (mg) | 100 | 100 |
| Thick Reduced Maltose Syrup | (mg) | 715 | 715 |
| Lactose | (mg) | 223 | 223 |
| Cacao Powder | (mg) | 78 | 78 |
| Flavor | (mg) | 19 | 19 |
| Fatty Acid Ester of Sucrose | (mg) | 65 | 65 |

Deposited Biological Materials

(1) Name and Addressee of Depository Authority

The International Patent Organism Depositary, National Institute of
Advanced Industrial Science and Technology
Tsukuba Central 6, 1-1, Higashi 1-chome Tsukuba-shi, Ibaraki-ken, Japan (Zip code 305-8566)

(2) Deposited Microorganisms

(i) *Alteromonas* sp. SN-1009

Original Date of Deposit: February 13, 1996
Date of Request for Transfer to International Deposit: November 15, 1996
Accession Number: FERM BP-5747

(ii) *Flavobacterium* sp. SA-0082

Original Date of Deposit: March 29, 1995
Date of Request for Transfer to International Deposit: February 15, 1996
Accession Number: FERM BP-5402

INDUSTRIAL APPLICABILITY

According to the present invention, there is provided an agent for maintaining homeostasis in a living body, comprising as an effective ingredient one or more members selected from the group consisting of fucoidan, a degradation product thereof and a salt thereof. The medicament exhibits an action for maintaining homeostasis in a living body by the above-mentioned effective ingredient, so that the medicament is useful as a therapeutic or prophylactic agent for hepatopathy, or an agent for maintaining homeostasis in blood. In addition, according to the present invention, there is provided a food, beverage or feed utilizing the action for maintaining homeostasis in a living body owned by the above-mentioned effective ingredient.

Furthermore, the present invention provides a fucoidan and a marine alga extract which are useful as raw materials for foods and the like; a process for preparing a fucoidan in high purity at high yield and a process for preparing a marine alga extract; and a food, beverage, seasoning, cosmetics and medicament, each comprising the fucoidan and/or the marine alga extract.

## Claims

1. An agent for maintaining homeostasis in a living body, **characterized in that** the agent comprises as an effective ingredient one or more members selected from the group consisting of fucoidan, a degradation product thereof and a salt thereof.

2. The agent according to claim 1, which is a therapeutic or prophylactic agent for a liver function disorder.

3. The agent according to claim 2, wherein the liver function disorder is a disease accompanying incidence of hepatic fibrosis.

4. The agent according to claim 1, which is an agent for maintaining homeostasis in blood.

5. The agent according to claim 4, having an suppressive action for elevating blood sugar level.

6. The agent according to claim 4, having an suppressive action for elevating neutral fat level in blood.

7. The agent according to any one of claims 4 to 6, having an action for maintaining homeostasis in blood after eating or drinking.

8. A food, beverage or feed for maintaining homeostasis in a living body, **characterized in that** the food, beverage or feed comprises one or more members selected from the group consisting of fucoidan, a degradation product thereof and a salt thereof.

9. The food, beverage or feed according to claim 8, which is usable for ameliorating or preventing a liver function disorder.

10. The food, beverage or feed according to claim 9, wherein the liver function disorder is a disease accompanying incidence of hepatic fibrosis.

11. The food, beverage or feed according to claim 8, which is a food, beverage or feed for maintaining homeostasis in blood.

12. The food, beverage or feed according to claim 11, having an suppressive action for elevating blood sugar level.

13. The food, beverage or feed according to claim 11, having an suppressive action for elevating neutral fat level in blood.

14. The food, beverage or feed according to any one-of claims 11 to 13, having an action for maintaining homeostasis in blood after eating or drinking.

15. A fucoidan obtainable by extracting a marine alga in the presence of a reducing substance.

16. The fucoidan according to claim 15, wherein the reducing substance is one or more reducing substances selected from the group consisting of ascorbic acid, ascorbates, erythorbic acid, erythorbates, cysteine and glutathione.

17. The fucoidan according to claim 15 or 16, wherein the extraction is carried out with hot water or a solvent.

**18.** The fucoidan according to any one of claims 15 to 17, wherein the extraction is carried out at 30° to 130°C for 5 minutes to 32 hours.

**19.** The fucoidan according to any one of claims 15 to 18, wherein the marine alga is previously treated with protease, and/or treated with protease in the extraction step.

**20.** The agent according to any one of claims 1 to 7, wherein the fucoidan is the fucoidan of any one of claims 15 to 19.

**21.** The food, beverage or feed according to any one of claims 8 to 14, wherein the fucoidan is the fucoidan of any one of claims 15 to 19.

**22.** A marine alga extract obtainable by extracting a marine alga in the presence of a reducing substance.

**23.** The marine alga extract according to claim 22, wherein the marine alga extract comprises a fucoidan.

**24.** The marine alga extract according to claim 22 or 23, wherein the reducing substance is one or more reducing substances selected from the group consisting of ascorbic acid, ascorbates, erythorbic acid, erythorbates, cysteine and glutathione.

**25.** The marine alga extract according to any one of claims 22 to 24, wherein the extraction is carried out with hot water or a solvent.

**26.** The marine alga extract according to any one of claims 22 to 25, wherein the extraction is carried out at 30° to 130°C for 5 minutes to 32 hours.

**27.** The marine alga extract according to any one of claims 22 to 26, wherein the marine alga is previously treated with protease, and/or treated with protease in the extraction step.

**28.** A food, beverage, seasoning or feed, **characterized in that** the food, beverage, seasoning or feed comprises the fucoidan of any one of claims 15 to 19 and/or the marine alga extract of any one of claims 22 to 27.

**29.** Cosmetics, **characterized in that** the cosmetics comprise the fucoidan of any one of claims 15 to 19 and/or the marine alga extract of any one of claims 22 to 27.

**30.** A medicament, **characterized in that** the medicament comprises the fucoidan of any one of claims 15 to 19 and/or the marine alga extract of any one of claims 22 to 27.

**31.** The medicament according to claim 30, which is a medicament for lowering cholesterol, a medicament for clarification of blood, a medicament for anti-coagulation, a medicament for anti-cancer, a medicament for anti-AIDS virus, or a medicament for anti-ulcer.

**32.** A process for preparing a fucoidan, **characterized in that** the process comprises the step of extracting a marine alga in the presence of a reducing substance.

**33.** The process according to claim 32, wherein the reducing substance is one or more reducing substances selected from the group consisting of ascorbic acid, ascorbates, erythorbic acid, erythorbates, cysteine and glutathione.

**34.** The process according to claim 32 or 33, wherein the extraction is carried out with hot water or a solvent.

**35.** The process according to any one of claims 32_to 34, wherein the extraction is carried out at 30° to 130°C for 5 minutes to 32 hours.

**36.** The process according to any one of claims 32 to 35, wherein the marine alga is previously treated with protease, and/or treated with protease in the extraction step.

**37.** A process for preparing a marine alga extract, **characterized in that** the process comprises the step of extracting a marine alga in the presence of a reducing substance.

**38.** The process according to claim 37, wherein the marine alga extract comprises a fucoidan.

**39.** The process according to claim 37 or 38, wherein the reducing substance is one or more reducing substances selected from the group consisting of ascorbic acid, ascorbates, erythorbic acid, erythorbates, cysteine and glutathione.

**40.** The process according to any one of claims 37 to 39, wherein the extraction is carried out with hot water or a solvent.

**41.** The process according to any one of claims 37 to 40, wherein the extraction is carried out at 30° to 130°C for 5 minutes to 32 hours.

**42.** The process according to any one of claims 37 to 41, wherein the marine alga is previously treated with protease, and/or treated with protease in the extraction step.

**43.** A process for manufacturing a food, beverage, seasoning or feed, **characterized in that** the process comprises the step in any one of claims 32 to 36 and/or the step in any one of claims 37 to 42.

**44.** A process for manufacturing cosmetics, **characterized in that** the process comprises the step in any one of claims 32 to 36 and/or the step in any one of claims 37 to 42.

**45.** A process for manufacturing a medicament, **characterized in that** the process comprises the step in any one of claims 32 to 36 and/or the step in any one of claims 37 to 42.

**46.** The process according to claim 45, which is a medicament for lowering cholesterol, a medicament for clarification of blood, a medicament for anti-coagulation, a medicament for anti-cancer, a medicament for anti-AIDS virus, or a medicament for anti-ulcer.

**47.** The fucoidan according to any one of claims 15 to 19, which is odorless or has reduced odor of the marine alga.

**48.** The fucoidan according to any one of claims 15 to 19, which is colorless or has reduced green-brown color derived from the marine alga.

**49.** The marine alga extract according to any one of claims 22 to 27, which is odorless or has reduced odor of the marine alga.

**50.** The marine alga extract according to any one of claims 22 to 27, which is colorless or has reduced green-brown color derived from the marine alga.

FIG.1

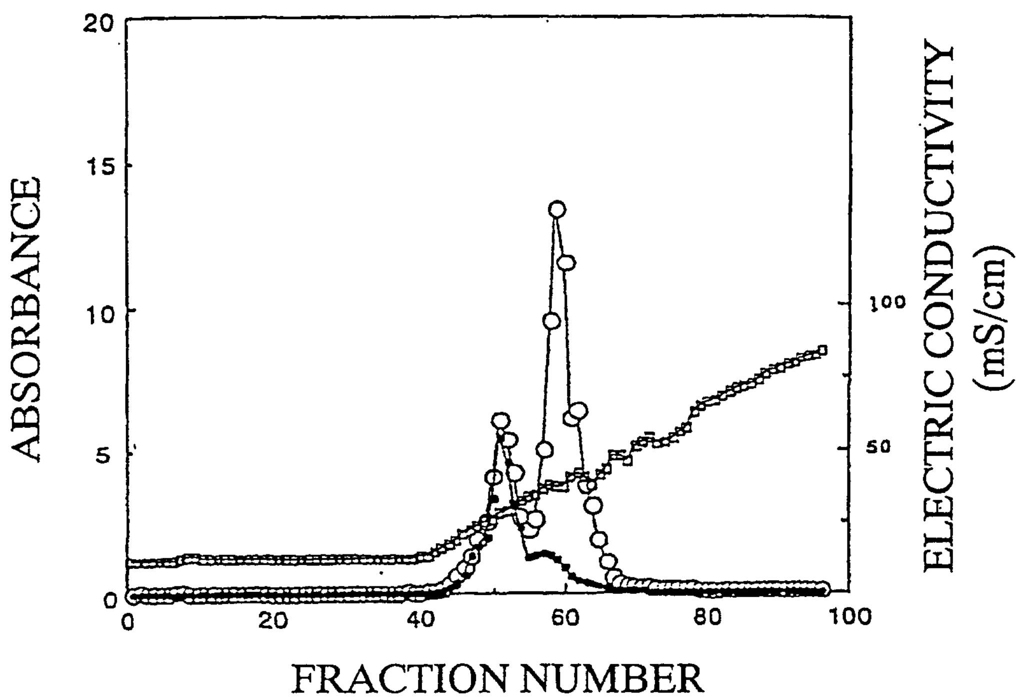
ABSORBANCE
0
5
10
15
20
ELECTRIC CONDUCTIVITY
(mS/cm)
50
100
0
20
40
60
80
100
FRACTION NUMBER

FIG.2

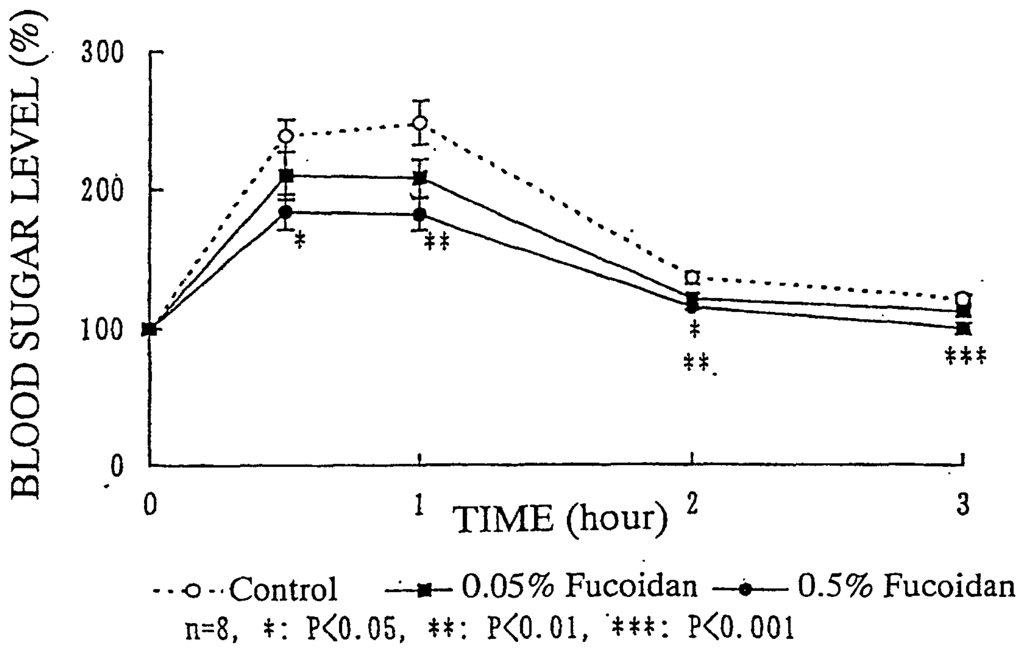
BLOOD SUGAR LEVEL (%)
300
200
100
0
0
1
TIME (hour)
2
3
*
**
*
**
***
--o--Control —■— 0.05% Fucoidan —●— 0.5% Fucoidan
n=8, *: P<0.05, **: P<0.01, ***: P<0.001

FIG.3

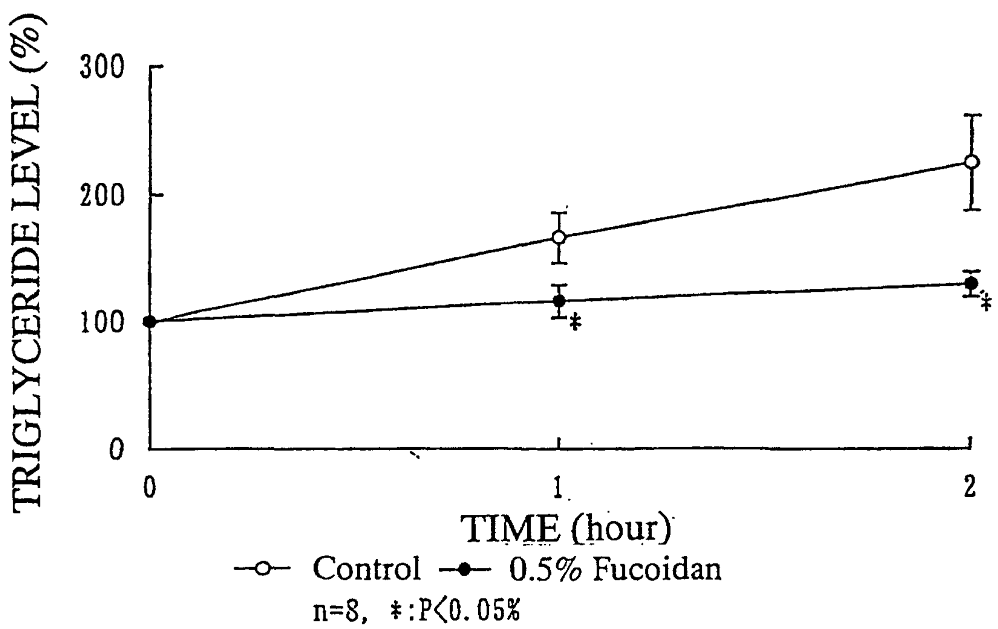
TRIGLYCERIDE LEVEL (%)
300
200
100
0
0
1
2
TIME (hour)
Control
0.5% Fucoidan
n=8, *:P<0.05%
*
*

# INTERNATIONAL SEARCH REPORT

International application No.
PCT/JP01/07894

A. CLASSIFICATION OF SUBJECT MATTER
Int.Cl$^7$ A61K31/737, 35/80, A61P43/00, 1/16, 3/08, 3/06, 35/00, 31/18, 1/04, A23L2/52, 1/29, A23K1/16, C08B37/00

According to International Patent Classification (IPC) or to both national classification and IPC

B. FIELDS SEARCHED

Minimum documentation searched (classification system followed by classification symbols)
Int.Cl$^7$ A61K31/737, 35/80, A61P43/00, 1/16, 3/08, 3/06, 35/00, 31/18, 1/04, A23L2/52, 1/29, A23K1/16, C08B37/00

Documentation searched other than minimum documentation to the extent that such documents are included in the fields searched

Electronic data base consulted during the international search (name of data base and, where practicable, search terms used)
CAPLUS (STN), MEDLINE (STN), EMBASE (STN), BIOSIS (STN), JICST (STN)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | WO 00/50464 A1 (Takara Shuzo Co., Ltd.), 31 August, 2000 (31.08.00), abstract; Claims; page 21, lines 14 to 18 & JP 2001-224369 A & JP 2001-226392 A | 1-7,15-20, 30,31,47,48<br>8-14,21-28, 32-46,49,50<br>29 |
| X<br>Y<br>A | KHOTIMCHENKO, Yu. S. et al., "The efficiency of dietary non-starch polysaccharides in experimental toxic hepatitis", Vopr. Pitan. January-April, 2000, Vol.69, No.1/2, pages 22 to 26 especially, abstract | 1-7,15-20, 30,31,47,48<br>8-14,21-29, 32-46,49,50<br>29 |
| X<br>Y<br>A | LI, Deyuan et al., "Effect of fucoidin on blood glucose in alloxan-induced diabetic mice", Huazhong Nongye Daxue Xuebao, (1999), Vol.18, No.2, pages 191 to 193, especially, abstract | 1-5,7,15-20, 30,47,48<br>8-14,21-29, 31-46,49,50<br>6,29 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 December, 2001 (11.12.01) | 25 December, 2001 (25.12.01) |
| Name and mailing address of the ISA/<br>Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP01/07894

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y<br><br>A | WO 99/41288 A1 (Takara Shuzo Co., Ltd.),<br>19 August, 1999 (19.08.99),<br>abstract; Claims; page 3, line 9 to page 7, line 8<br>& EP 1057833 A1 & JP 2001-261704 A<br>& AU 9924401 A1 | 1-4,6,15-20,<br>29-31,47,48<br>8-14,21-28,<br>32-46,49,50<br>5 |
| X<br><br>Y | EP 916269 A1 (Takara Shuzoh Co., Ltd.),<br>19 May, 1999 (19.05.99),<br>abstracts; Claims; Par. No. [0020]<br>& WO 97/47208 A1 & AU 9727898 A1<br>& AU 711896 B2 & CN 1221320 A<br>& KR 2000010670 A | 15-19,28,47,48<br><br>21-27,43 |
| X | EP 919237 A1 (Takara Shuzoh Co., Ltd.),<br>02 June, 1999 (02.06.99),<br>the whole document<br>& WO 97/26896 A1 & US 6207652 B1<br>& US 2001034335 A1 & JP 2001-218580 A<br>& JP 2001-226407 A & JP 2001-226408 A<br>& JP 2001-224394 A2 & AU 9673555 A1<br>& CA 2243543 A & AU 9713999 A1<br>& AU 720004 B2 & CN 1209749 A | 15-19,47,48 |
| Y<br>A | GB 2219484 A (Yoshio TANAKA),<br>13 December, 1989 (13.12.89),<br>Claims<br>& DE 3905866 A & JP 1-218573 A<br>& FR 2627672 A & AU 8822122 A<br>& US 4913915 A & GB 2219484 B<br>& DE 3905866 C & IL 87261 A<br>& IT 1228458 A & JP 4-75753 B<br>& CH 681416 A | 22-46,49,50<br>15-21 |
| Y<br>A | JP 8-66159 A (Miho TANAKA),<br>12 March, 1996 (12.03.96),<br>Par. Nos. [0003], [0004]<br>(Family: none) | 22-46,49,50<br>15-21 |
| Y<br>A | JP 61-57519 A (Kabushiki Kaisha Kibun Food Chemiphar.),<br>24 March, 1986 (24.03.86),<br>Claims<br>(Family: none) | 22-46,49,50<br>15-21 |
| Y<br>A | JP 61-57520 A (Kabushiki Kaisha Kibun Food Chemiphar.),<br>24 March, 1986 (24.03.86),<br>Claims<br>(Family: none) | 22-46,49,50<br>15-21 |
| PX<br><br><br>PA | WO 01/13925 A1 (Takara Shuzo Co., Ltd.),<br>01 March, 2001 (01.03.01),<br>abstract<br>(Family: none) | 15-19,21,47,48<br><br><br>1-14,20 |
| PX | WO 00/62785 A1 (Takara Shuzo Co., Ltd.),<br>26 October, 2000 (26.10.00),<br>abstract; Claims<br>(Family: none) | 15-19,21,<br>28-30,47,48 |

Form PCT/ISA/210 (continuation of second sheet) (July 1992)

INTERNATIONAL SEARCH REPORT

International application No.
PCT/JP01/07894

C (Continuation). DOCUMENTS CONSIDERED TO BE RELEVANT

| Category* | Citation of document, with indication, where appropriate, of the relevant passages | Relevant to claim No. |
|---|---|---|
| PX | LI, Fuchuan et al., "Hypoglycemic activity of 3 polysaccharides from Laminaria japonica", Zhongguo Haiyang Yaowu, (2000), Vol.19, No.5, pages 12 to 15, especially, abstract | 1-5,7,15-20, 30,47,48 |

Form PCT/ISA/210 (continuation of second sheet) (July 1992)

**INTERNATIONAL SEARCH REPORT**

International application No.
PCT/JP01/07894

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The technical feature of claims 1 to 21, 47 and 48 resides in fucoidan and a specific use of fucoidan, while the technical feature of claims 22 to 46, 49 and 50 resides in a method of extracting marine algae and the extraction method. Namely, these technical features are neither identical to each other nor correspond to each other. Such being the case, these inventions are not considered as relating to a group of inventions so linked as to form a single general inventive concept.

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)